# EUROPEAN PATENT APPLICATION

(11) **EP 3 988 676 A1**
(43) Date of publication of application: **27.04.2022**
(21) Application number: 20203612.5
(22) Date of filing: 23.10.2020
(51) Int. Cl.: C12Q 1/689

(54) **MULTIPLEX PCR FOR DETECTION OF CARBAPENEMASE GENES**

(71) Applicant: Universität Heidelberg, 69117 Heidelberg (DE); FRIZ Biochem GmbH, 82061 Neuried (DE)
(72) Inventor: PROBST, Katja, 69151 Neckargemünd (DE); DALPKE, Alexander, 01237 Dresden (DE); HEEG, Klaus, 69126 Heidelberg (DE); BANDILLA, Michael, 82234 Wessling (DE)
(74) Representative: Zwicker, Jörk

(57) **Abstract**

The present invention relates to a method for determining in a sample the presence or absence of carbapenemase producing bacteria, comprising determining the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene. In addition, the invention relates to an oligonucleotide set and a kit suitable for use in the claimed method. The invention further relates to a method of diagnosis of an infection with carbapenemase producing bacteria, a method for selecting a patient for treatment with a beta lactam antibacterial drug and a beta lactam antibacterial drug, preferably a carbapenem, for use in a method of treatment of a bacterial infection in a patient that is not infected with carbapenemase producing bacteria.

## Description

### FIELD OF THE INVENTION

The present invention relates to the field of bacterial resistance, in particular to resistance against beta lactam antibacterial drugs, more particularly to detection of bacteria producing carbapenemase genes by multiplex PCR.

### BACKGROUND OF THE INVENTION

Resistance to beta-lactam antibiotics in gram-negative bacilli is an increasing problem worldwide. In case of resistance to carbapenems, the remaining treatment options for severe infections are limited (Kaase 2012). Carbapenem resistance occurs mostly via carbapenemases, acquired enzymes that are able to hydrolyze different beta-lactam antibiotics, but also through a combination of porin loss or overexpression of efflux pumps with expression of extended-spectrum beta lactamases (ESBLs) or AmpCs, depending on the bacteria. Carbapenemases are usually located on plasmids that allow facilitated spreading. The first carbapenemase (NmcA) was discovered in an *Enterobacteriaceae* in 1993 (Naas and Nordmann 1994). Over the last years, a large variety of carbapenemases have been described but the most important remain KPC, NDM, VIM and OXA-48. These four enzymes are also the most prevalent carbapenemases found in *Enterobacteriaceae* over the last years in Germany (Koch-Institut 2016; Pfennigwerth 2017; Koch-Institut 2015; Pfennigwerth 2018). In the non-fermenters *Pseudomonas aeruginosa* and *Acinetobacter baumannii* VIM, IMP and OXA-23, OXA-72 and OXA-58, respectively, were detected more frequently (ibd.).

Carbapenemases are classified based on their amino acid sequence into different Ambler classes. KPC, a serine beta-lactamase, belongs to Ambler class A, NDM, VIM and IMP, also known as metallo-beta-lactamases, belong to class B, and the oxacillinases OXA-48, OXA-40/24, OXA-23 and OXA-58 classify in class D. Numerous variants exist within each class due to mutations of the genes. The number of carbapenemase variants is steadily increasing.

For infection control, detection methods that cover different carbapenemases and their variants are needed. A rapid identification and differentiation of carbapenemases in the clinical setting is important to define suitable antibiotic therapies, take hygienic measures and be able to counteract further spread. Besides phenotypic detection methods of carbapenem resistance, a number of different genotypic assays, based on monoplex or multiplex quantitative real-time PCR and using hydrolysis probes or melt-curve analysis have been developed (e.g. Hofko et al. 2014). Examples are Eazyplex^{®} SuperBug CRE assay (Amplex Biosystems GmbH, Gießen, Germany), the Check-Direct CPE assay (Check-Points, Wageningen, The Netherlands) or the Cepheid Xpert Carba-R assay (Cepheid, Sunnyvale, CA, USA). Some assays include certain IMP variants, different extended spectrum beta-lactamases (ESBLs) and AmpC beta-lactamases (AmpCs). However, most of the commercially available assays are limited to the "big 4" carbapenemases commonly found in *Enterobacteriaceae* (KPC, NDM, VIM and OXA-48). Even of KPC, NDM, VIM and OXA-48, not all recently occurring gene variants are included. The carbapenemases IMP and OXA occurring in the nonfermenters *Pseudomonas aeruginosa* and *Acinetobacter baumannii*, which constitute a significant proportion of detected carbapenemases in Germany, are only partially covered or not covered at all.

Here, the inventors performed a careful analysis of recently occurring carbapenemase genes to define new primers and probes for the detection of carbapenemases in *Enterobacteriaceae* and in the non-fermenters *P. aeruginosa* and *A. baumannii.* The primers and probes are suitable to be used in a multiplex PCR approach. Thus, the present inventors have overcome one or more of the above stated problems of the prior art. The carbapenemase detection assay of the present invention provides inter alia one or more of the following advantages: (i) detection 97.1% of the carbapenemases reported by the German National Reference Laboratory (NRL); (ii) detection of recently occurring carbapenemase gene variants; (iii) detection of multiple IMP carbapenemase genes, including IMP-7, -13, -14, -15 and -28; (iv) detection of OXA-40/24-like and OXA-58-like carbapenemase genes; (v) simultaneous detection of NDM, KPC, VIM, IMP, OXA-23-like, OXA-48-like, OXA-58-like and OXA-40/24-like carbapenemase genes including most known variants; (vi) increased sensitivity; (vii) increased specificity; (viii) decreased time needed for carbapenemase gene detection; (ix) decreased limit of detection (LOD) for single carbapenemase genes; (x) easy realization and implementation of the assay; (xi) suitability of the assay for automatization.

### SUMMARY OF THE INVENTION

In a first aspect, the present invention relates to a method for determining in a sample the presence or absence of carbapenemase producing bacteria, the method comprising determining in said sample the presence or absence of one or more carbapenemase genes selected from the group consisting of
a) an NDM carbapenemase gene, the presence of which is determined using a forward primer F1, a reverse primer R1 and optionally a probe P1, wherein F1, R1 and P1 are each capable of hybridization to SEQ ID NO: 1 or its complement;
b) a KPC carbapenemase gene, the presence of which is determined using a forward primer F2, a reverse primer R2 and optionally a probe P2, wherein F2, R2 and P2 are each capable of hybridization to SEQ ID NO:27 or its complement;
c) a VIM carbapenemase gene, the presence of which is determined using a forward primer F3, a reverse primer R3 and optionally a probe P3, wherein F3, R3 and P3 are each capable of hybridization to SEQ ID NO:47 or its complement;
d) an IMP carbapenemase gene, the presence of which is determined using a forward primer F4, a reverse primer R4 and optionally a probe P4, wherein F4, R4 and P4 are each capable of hybridization to SEQ ID NO:69 or its complement, SEQ ID NO:79 or its complement, or SEQ ID NO:86 or its complement;
e) an OXA-23-like carbapenemase gene, the presence of which is determined using a forward primer F5, a reverse primer R5 and optionally a probe P5, wherein F5, R5 and P5 are each capable of hybridization to SEQ ID NO:93 or its complement;
f) an OXA-48-like carbapenemase gene, the presence of which is determined using a forward primer F6, a reverse primer R6 and optionally a probe P6, wherein F6, R6 and P6 are each capable of hybridization to SEQ ID NO:113 or its complement;
g) an OXA-58-like carbapenemase gene, the presence of which is determined using a forward primer F7, a reverse primer R7 and optionally a probe P7, wherein F7, R7 and P7 are each capable of hybridization to SEQ ID NO:127 or its complement; and
h) an OXA-40/24-like carbapenemase gene, the presence of which is determined using a forward primer F8, a reverse primer R8 and optionally a probe P8, wherein F8, R8 and P8 are each capable of hybridization to SEQ ID NO:138 or its complement.

In a second aspect, the present invention relates to an oligonucleotide set comprising
a) a forward primer F1 comprising or consisting of SEQ ID NO:15, a reverse primer R1 comprising or consisting of SEQ ID NO:16 and optionally a probe P1 comprising or consisting of SEQ ID NO:17;
b) a forward primer F2 comprising or consisting of SEQ ID NO:38, a reverse primer R2 comprising or consisting of SEQ ID NO:39 and optionally a probe P2 comprising or consisting of SEQ ID NO:40;
c) a forward primer F3 comprising or consisting of SEQ ID NO:59, a reverse primer R3 comprising or consisting of SEQ ID NO:60 and optionally a probe P3 comprising or consisting of SEQ ID NO:61;
d) a forward primer F4 comprising or consisting of SEQ ID NO:73, a reverse primer R4 comprising or consisting of SEQ ID NO:74 and optionally a probe P4 comprising or consisting of SEQ ID NO:75;
e) a forward primer F5 comprising or consisting of SEQ ID NO:104, a reverse primer R5 comprising or consisting of SEQ ID NO:105 and optionally a probe P5 comprising or consisting of SEQ ID NO:106;
f) a forward primer F6 comprising or consisting of SEQ ID NO:121, a reverse primer R6 comprising or consisting of SEQ ID NO:122 and optionally a probe P6 comprising or consisting of SEQ ID NO:123;
g) a forward primer F7 comprising or consisting of SEQ ID NO:133, a reverse primer R7 comprising or consisting of SEQ ID NO:134 and optionally a probe P7 comprising or consisting of SEQ ID NO:135; and/or
h) a forward primer F8 comprising or consisting of SEQ ID NO:143, a reverse primer R8 comprising or consisting of SEQ ID NO:144 and optionally a probe P8 comprising or consisting of SEQ ID NO:145.

In a third aspect, the present invention relates to a kit comprising an oligonucleotide set according to the second aspect of the invention and a component selected from the group consisting of a buffer, nucleotides and a DNA polymerase.

In a fourth aspect, the present invention relates to a use of the oligonucleotide set according to second aspect or the kit according the third aspect for determining in a sample the presence or absence of carbapenemase producing bacteria.

In a fifth aspect, the present invention relates to a method of diagnosis of an infection with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene; wherein the presence of a carbapenemase gene in the sample is indicative of an infection with carbapenemase producing bacteria.

In a sixth aspect, the present invention relates to a method of treatment of a bacterial infection, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample comprises carbapenemase producing bacteria, administering to the patient an antibacterial drug different from a beta lactam antibacterial drug, preferably different from a carbapenem, preferably an antibacterial drug selected from the group consisting of colistin, gentamicin, ceftazidime-avibactam, ceftolozane-tazobactam, fosfomycin, nitrofurantoin, pivmecillinam and temocillin; if the sample does not comprise carbapenemase producing bacteria, administering to the patient a beta lactam antibacterial drug, preferably a carbapenem.

In a seventh aspect, the present invention relates to an in vitro method for selecting a patient for treatment with a beta lactam antibacterial drug, preferably a carbapenem, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, selecting the patient for treatment with a beta lactam antibacterial drug, preferably a carbapenem.

In an eighth aspect, the present invention relates to a beta lactam antibacterial drug, preferably a carbapenem for use in a method of treatment of a bacterial infection in a patient that is not infected with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, administering to the patient a beta lactam antibacterial drug, preferably a carbapenem.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturers' specifications, instructions etc.), whether supra or infra, is hereby incorporated by reference in its entirety.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. In preferred embodiments, "comprise" can mean "consist of". As used in this specification and the appended claims, the singular forms "a", "an", and "the" include plural referents, unless the content clearly dictates otherwise.

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

In a first aspect, the present invention relates to a method for determining in a sample the presence or absence of carbapenemase producing bacteria, the method comprising determining in said sample the presence or absence of one or more carbapenemase genes selected from the group consisting of
a) an NDM carbapenemase gene, the presence of which is determined using a forward primer F1, a reverse primer R1 and optionally a probe P1, wherein F1, R1 and P1 are each capable of hybridization to SEQ ID NO:1 or its complement;
b) a KPC carbapenemase gene, the presence of which is determined using a forward primer F2, a reverse primer R2 and optionally a probe P2, wherein F2, R2 and P2 are each capable of hybridization to SEQ ID NO:27 or its complement;
c) a VIM carbapenemase gene, the presence of which is determined using a forward primer F3, a reverse primer R3 and optionally a probe P3, wherein F3, R3 and P3 are each capable of hybridization to SEQ ID NO:47 or its complement;
d) an IMP carbapenemase gene, the presence of which is determined using a forward primer F4, a reverse primer R4 and optionally a probe P4, wherein F4, R4 and P4 are each capable of hybridization to SEQ ID NO:69 or its complement, SEQ ID NO:79 or its complement, or SEQ ID NO:86 or its complement;
e) an OXA-23-like carbapenemase gene, the presence of which is determined using a forward primer F5, a reverse primer R5 and optionally a probe P5, wherein F5, R5 and P5 are each capable of hybridization to SEQ ID NO:93 or its complement;
f) an OXA-48-like carbapenemase gene, the presence of which is determined using a forward primer F6, a reverse primer R6 and optionally a probe P6, wherein F6, R6 and P6 are each capable of hybridization to SEQ ID NO:113 or its complement;
g) an OXA-58-like carbapenemase gene, the presence of which is determined using a forward primer F7, a reverse primer R7 and optionally a probe P7, wherein F7, R7 and P7 are each capable of hybridization to SEQ ID NO:127 or its complement; and
h) an OXA-40/24-like carbapenemase gene, the presence of which is determined using a forward primer F8, a reverse primer R8 and optionally a probe P8, wherein F8, R8 and P8 are each capable of hybridization to SEQ ID NO:138 or its complement.

The term "determining" as used herein refers to at least qualitatively analysing for the presence or absence of a carbapenemase gene. "At least qualitatively" means that also a quantitative analysis of the amount of a carbapenemase gene, if present, can be performed. Such a "determining the amount" can be performed as described herein. Generally, the quantification may be absolute, e.g. in copies per µl sample, or it may be relative, e.g. 10-fold higher than in a control sample or as percentage of the amount of a reference gene. Determining the absolute amount of a carbapenemase gene in the sample may comprise normalizing for the amount of total DNA in the sample and the average genome size of the bacterial species. In case of determining a relative amount as percentage of the amount of a reference gene a reference gene, the reference gene is preferably a housekeeping gene.

A housekeeping gene is a constitutively expressed gene involved in or required for the maintenance of basic cellular function and is expected to always be available in constant amounts. An example for a bacterial reference gene is the 16S rDNA gene.

Quantitative analysis enables to identify samples comprising carbapenemase producing bacteria having a clinically significant carbapenemase-producing activity. The term "bacteria having a clinically significant carbapenemase producing activity" refers to bacteria showing positive carbapenemase activity in a clinically recommended phenotypic carbapenemase production test including, but not limited to, the VITEK 2 system (bioMérieux Deutschland GmbH, Nürtingen, Germany).

Whenever it is mentioned in the present specification that the presence of a carbapenemase gene or the presence of carbapenemase producing bacteria is determined, this is meant to include determination of the absence of the carbapenemase gene or the carbapenemase producing bacteria.

The method according to the first aspect of the invention involves amplification and/or detection of carbapenemase genes present in bacterial genomic DNA. As used herein, the term "bacterial genomic DNA" encompasses the different types of DNA that may be present in a bacterial cell, including plasmid DNA and chromosomal DNA. The methods according to the invention allow the detection of a low number of copies of bacterial genomic DNA. In certain embodiments, the methods described herein are sufficiently sensitive to allow the detection of a reduced number of copies of bacterial genomic DNA. In embodiments, the detection methods described herein provide for detection of as low as less than 100 copies, or as low as less than 50 copies, or as low as less than 25 copies, or as low as less than 10 copies, or as low as less than 5 copies, or as low as only one copy of bacterial genomic DNA.

The inventors have shown that the method according to the invention results in a decreased limit of detection (LOD) compared to other methods know in the art. Using the method according to the invention, the LOD was as low as 3 copies per PCR (e.g. for NDM, VIM and OXA-like carbapenemase genes).

In the context of the present specification, the term "sample" includes a direct clinical sample (e.g. a biological specimen obtained from bodily fluids such as blood or urine, or throat swabs, nasal swabs, rectal swabs, dermal swabs, sputum, feces, bronchial aspirates, etc.) as well as a processed specimen (e.g. a clinical isolate obtained following bacterial culture or purified nucleic acids). The term also encompasses bacterial cultures from various environments as well as various environmental samples that may originate from, but not limited to, hospitals (e.g. swabs of laboratory working surfaces, swabs of medical instruments, swabs of a patient room, etc.), public spaces (e.g. swabs of an object from school, shopping malls, etc.), nature (e.g. water, air, soil, etc.), and the like. Methods for deriving samples from a subject or the environment are well known to those skilled in the art.

In the context of the present specification, the term "carbapenem" refers to an antibacterial drug of the class of beta-lactam antibacterial drugs. Carbapenems are very similar to penicillins, wherein the sulphur atom at position 1 of the beta-lactam ring is replaced with an unsubstituted carbon atom. Carbapenems are often used for the treatment of severe or high-risk bacterial infections and are usually reserved for known or suspected multidrug-resistant (MDR) bacterial infections. Examples of carbapenems are imipenem, meropenem, ertapenem, doripenem, panipenem, biopenem and tebipenem.

In the context of the present specification, the term "carbapenemase" refers to an enzyme carbapenem-hydrolyzing activity. In addition, carbapenemases are capable of hydrolyzing penicillins, cephalosporins, monobactams. Carbapenemases are members of the Ambler classes A, B, and D of β-lactamases. Class A and D enzymes have a serine-based hydrolytic mechanism, while class B enzymes are metallo-β-lactamases that contain zinc in the active site. In class A, the KPC carbapenemases are the most prevalent group, found mostly on plasmids in *Klebsiella pneumoniae.* The class D carbapenemases consist of OXA-type β-lactamases frequently detected in *Acinetobacter baumannii.* The metallo-β-lactamases of class B belong to the IMP, VIM, SPM, GIM, and SIM families and have been detected primarily in *Pseudomonas aeruginosa.*

Since carbapenemase producing bacteria are able to hydrolyze carbapenems and several other beta-lactam anti-bacterial drugs, carbapenemase producing bacteria resistant or at least less susceptible to treatment with carbapenems and other beta-lactam anti-bacterial drugs.

The term "NDM carbapenemase" includes any carbapenemase having at least 85%, at least 90%, at least 95%, at least 96%, at least 97%, at least 98%, at least 99% or more sequence identity (at the amino acid or nucleic acid level) with an NDM carbapenemase of accession number FN396876, JF703135, JQ734687, JQ348841, JN104597, JN967644, JX262694, AB744718, KC999080, KF361506, AB926431, LC012596, NG_049328.1, NG_049332.1, NG_049333.1, KX812714.1, Y503030.1, MF370080.1 or MG183694.1.

The term "KPC carbapenemase" includes any carbapenemase having at least 85%, at least 90%, at least 95%, at least 96%, at least 97%, at least 98%, at least 99% or more sequence identity (at the amino acid or nucleic acid level) with a KPC carbapenemase of accession number AF297554, AY034847, HM769262, FJ473382, EU400222, EU555534, EU729727, FJ234412, FJ624872, GQ140348, HM066995, HQ342889, HQ342890, JX524191, KC433553, KC465199, NG_049249.1, NG_049250.1, NG_049251.1, NG_049252.1, MF772496.1, NG_049254.1, NG_049255.1, NG_051167.1, NG_051469.1, NG_052862.1, KY282958.1, KY563764.1, KY646302.1, NG_055494.1 or NG_055495.1.

The term "VIM carbapenemase" includes any carbapenemase having at least 85%, at least 90%, at least 95%, at least 96%, at least 97%, at least 98%, at least 99% or more sequence identity (at the amino acid ornucleic acid level) with a VIM carbapenemase of accession number Y18050, AF302086, AF300454, EU581706, DQ023222, AY165025, AJ536835, AY524987, AY524988, AY524989, AY605049, DQ143913, DQ365886, FJ445404, EU419745, EU419746, EU118148, AM778091, FJ499397, GQ414736, GQ242167, HM855205, HM750249, FR748153, HQ858608, JF900599, JX311308, JN129451, JN982330, JN676230, JX258134, JX013656, JX982634, JX982635, JX982636, KC469971, NG_050379.1, NG_050374.1, KY508061.1, HG934765.1, KT964061.1, KX349731.1, NG_050370.1, NG_050366.1, KP771862.1, KU663375.1, KU663374.1, KT954134.1, NG_050371.1, NG_050372.1 or NG_050373.1.

The term "IMP carbapenemase" includes any carbapenemase having at least 85%, at least 90%, at least 95%, at least 96%, at least 97%, at least 98%, at least 99% or more sequence identity (at the amino acid or nucleic acid level) with an IMP carbapenemase of accession number DQ522237, AJ243491, AB010417, AF244145, DQ307573, JF810083, AB188812, AF318077, EU042136, AY033653, AB195637, AB074436, AJ420864, AJ512502, AY553332, EF184216, AJ584652, EF184215, EF118171, AB196988, DQ361087, EF192154, HM175876, GU045307, JF894248, JQ407409, HQ438058, KF148593, JQ002629, JN848782, AB715422, JF816544, JX131372, HQ875573, AB753457, AB753458, AB753456, AB777500, AB777501, NG_049180.1, NG_049185.1, NG_049187.1, NG_049195.1, NG_049209.1, NG_049210.1, NG_049211.1, NG_049213.1, NG_049214.1, NG_049215.1, KU052795.1, KU299753.1, KU315553.1, KU647281.1, KX196782.1, NG_050945.1, KX462700.1, KX753224.1, KX821663.1, NG_052050.1, LC190726.1, MF281100.1, MF669572.1 or MF678349.1.

The term "OXA-23-like carbapenemase" includes any carbapenemase having at least 85%, at least 90%, at least 95%, at least 96%, at least 97%, at least 98%, at least 99% or more sequence identity (at the amino acid or nucleic acid level) with an OXA-23-like carbapenemase of accession number EU571228, FJ194460, HM488986, HM488987, HM488988, HM488989, HM488990, HM488991, HM488992, JN638887, HQ700358, JQ837239, AF201828, AY288523, KY883665.1 or AY762325.

The term "OXA-48-like carbapenemase" includes any carbapenemase having at least 85%, at least 90%, at least 95%, at least 96%, at least 97%, at least 98%, at least 99% or more sequence identity (at the amino acid or nucleic acid level) with an OXA-48-like carbapenemase of accession number GU197550, HQ700343, HM992946, JQ809466, JX423831, JX438000, JX438001, JX893517, KF900153.1, AY236073, NG_055475.1, NG_054666.1, NG_055490.1, NG_052051.1 or NG_054693.1.

The term "OXA-58-like carbapenemase" includes any carbapenemase having at least 85%, at least 90%, at least 95%, at least 96%, at least 97%, at least 98%, at least 99% or more sequence identity (at the amino acid or nucleic acid level) with an OXA-58-like carbapenemase of accession number GU831575, KU726870.1, AY665723, DQ519090 or EF102240.

The term "OXA-40/24-like carbapenemase" includes any carbapenemase having at least 85%, at least 90%, at least 95%, at least 96%, at least 97%, at least 98%, at least 99% or more sequence identity (at the amino acid or nucleic acid level) with an OXA-40/24-like carbapenemase of accession number AM991978, GU199038, JQ838185, AJ239129, AF201826, AF201827, GU199039, GQ861437, HM640278, JQ326200, KC479324 or KC479325.

Each of the eight carbapenemase families listed above (NDM, KPC, VIM, IMP, OXA-23-like, OXA-48-like, OXA-58-like and OXA-40/24-like) comprises many variants. The IMP family e.g. comprises IMP-42, IMP-60, IMP-6, IMP-30, IMP-66, IMP-10 and many more. The OXA-48-like family e.g. comprises OXA-514, OXA, 547, OXA-519 and many more. As used herein, the term "variant" refers to an enzyme which differs by one or more amino acids from the first protein sequence described and characterized for each carbapenemase family, wherein the difference may be a substitution, a deletion, or an insertion (Widmann M et al., 2012, Antimicrobial Agents & Chemotherapy 56:3481-3491).

In the context of the present specification, the term "hybridization", when used with respect to an oligonucleotide, is meant to refer to specific hybridization and is to be understood as a bond of an oligonucleotide to an at least substantially complementary sequence along the lines of the Watson-Crick base pairings in the sample DNA, forming a duplex structure, under moderate or stringent hybridization conditions. When it is used with respect to a single nucleotide or base, it refers to the binding according to Watson-Crick base pairings, e.g. C-G, A-T and A-U. "Substantially complementary" means that an oligonucleotide does not need to reflect the exact sequence of the template (i.e. the target sequence) and can comprise mismatches as defined below. In preferred embodiments, the primers and the probes capable of hybridization to a target sequence comprise a nucleotide sequence having at least 85%, at least 90%, at least 93% or at least 95% identity with said target sequence.

Stringent hybridization conditions involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderate conditions involve washing in 3x SSC at 42°C, or the art-recognized equivalent thereof. The parameters of salt concentration and temperature can be varied to achieve the optimal level of hybridization strengh between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The term "mismatch" as used herein refers to base-pair mismatch in DNA, more specifically a base-pair that is unable to form normal base-pairing interactions (i.e., other than "A" with "T" or "U", or "G" with "C").

In the context of the present specification, the IUPAC for nucleotides is used. According to this nomenclature, A refers to adenine, C refers to cytosine, G refers to guanine, T refers to thymine, U refers to uracil, W (weak) refers to A and T, S (strong) refers to C or G, K (keto) refers to G or T, M (amino) refers to A or C, Y (pyrimidine) refers to C or T, R (purine) refers to A or G, B refers to C, G or T, D refers to A, G or T, H refers to A, C or T, V refers to A, C or G and N refers to A, C, G or T.

The term "oligonucleotide" as used herein refers to a linear oligomer of 5 to 50 ribonucleotides or preferably deoxyribonucleotides. Preferably, it has the structure of a single-stranded DNA fragment.

The term "primer oligonucleotide" or "primer" as used herein refers to a single-stranded oligonucleotide substantially complementary to a nucleic acid sequence sought to be amplified (the template) and serves as a starting point for synthesis of a primer extension product.

In preferred embodiments of the first aspect of the invention, the primers are characterized by a GC content of 35-65%; a length of 17-30 bp; and/or a melting temperature of 57-62 °C, preferably 58-61 °C. The skilled person is well capable of determining the melting temperature of a primer oligonucleotide. An exemplary method is described in the methods section below. Preferably, the primers are characterized by a GC content of 35-65%; a length of 17-30 bp; and a melting temperature of 58-61 °C.

It is preferred that the presence or absence of a carbapenemase gene according to the method of the first aspect of the invention is determined using a forward primer, a reverse primer and a probe.

The term "probe oligonucleotide" or "probe" as used herein refers to a single-stranded oligonucleotide substantially complementary to a nucleic acid sequence sought to be amplified (the template) and serves to detect an amplicon by annealing to one strand of the amplicon, usually not where any of the primer oligonucleotides bind (i.e. not to a sequence segment of the one strand which overlaps with a sequence segment a primer oligonucleotide anneals to). The probe may be linked, preferably covalently linked, to at least one detectable label which allows detection of the amplicon. The term "detectable label" as used herein does not exhibit any particular limitation. The detectable label may be selected from the group consisting of radioactive labels, luminescent labels, fluorescent dyes, compounds having an enzymatic activity, magnetic labels, antigens, and compounds having a high binding affinity for a detectable label. For example, fluorescent dyes linked to a probe may serve as a detection label, e.g. in a real-time PCR. Suitable radioactive markers are P-32, S-35, 1-125, and H-3, suitable luminescent markers are chemiluminescent compounds, preferably luminol, and suitable fluorescent markers are preferably dansyl chloride, fluorcein-5-isothiocyanate, and 4-fluor-7-nitrobenz-2-aza-1,3 diazole, in particular 6-carboxyfluorescein (FAM), 6-hexachlorofluorescein (HEX), 5(6)-carboxytetramethylrhodamine (TAMRA), 5(6)-carboxy-X-Rhodamine (ROX), cyanin-5-fluorophor (Cy5) and derivates thereof; suitable enzyme markers are horseradish peroxidase, alkaline phosphatase, a-galactosidase, acetylcholinesterase, or biotin. In some embodiments, the probe is linked to a fluorescent dye and a corresponding quencher, wherein one is located at the 3'-end and the other at the 5'-end. In such embodiments, a fluorescent signal emitted from the fluorescent dye is emitted upon hydrolysis of the probe during the primer elongation steps of a PCR. Alternatively, probes may comprise detection moieties that enable their electrochemical detection in a microelectrode array (e.g. for use in the CYCLE^{®} Technology developed by FRIZ Biochem).

In preferred embodiments of the first aspect of the invention, the probes are characterized by a GC content of 40-60%; a length of 20-40 bp, preferably 20-30 bp; and/or a melting temperature of 68-72 °C. Preferably, the probes are characterized by a GC content of 40-60%; a length of 20-40 bp, preferably 20-30 bp; and a melting temperature of 68-72 °C.

The primers and probes show a high specificity towards their respective carbapenemase genes, which was confirmed by Primer BLAST against the non-redundant database and the collected beta-lactamase resistance genes. The primers and probes do not show hairpin formation or formation of self-/heterodimers. For use in multiplex PCR, the primers and probes were selected such that no multimers, in particular dimers, of primers and/or probes will form. Preferably, the primers and probes show a change in free energy (ΔG) of less than -9.0 kcal/mol for hairpin and multimer formation. The primers and probes do not comprise single nucleotide repetitions of more than 5 nucleotides, preferably not more than 4 nucleotides, more preferably not more than 3 nucleotides.

The primers and probes may comprise a mismatch with respect to the target sequence. In a preferred embodiment, the number of mismatches is 3 or less, more preferably 2 or less and most preferably 1 or less.

In a preferred embodiment, all mismatches are in the middle third or 5' third of a primer oligonucleotide.

In some embodiments, the expression "method for determining in a sample the presence or absence of carbapenemase producing bacteria" as used herein refers to diagnosis of an infection with carbapenemase producing bacteria in a patient of which the sample is obtained. As will be understood by persons skilled in the art, such assessment normally may not be correct for 100% of the patients, although it preferably is correct. The term, however, requires that a correct indication can be made for a statistically significant part of the subjects. Whether a part is statistically significant can be determined easily by the person skilled in the art using several well-known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%, at least 95%. The p values are preferably 0.05, 0.01, or 0.005.

In the context of the present specification, the term "sensitivity" relates to the percentage of samples containing carbapenemase producing bacteria that were correctly identified.

In the context of the present specification, the term "specificity" relates to the percentage of samples that were correctly identified as not containing carbapenemase producing bacteria.

The term "subject" as used herein refers to an individual, such as a human, a non-human primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a particular meaning, the subject is a mammal. In a preferred meaning, the subject is a human.

It can be envisioned that the method according to the first aspect of the invention comprises the steps of (a) amplification of a region of bacterial DNA comprised in the sample using at least one primer pair consisting of a forward and a corresponding reverse primer recited for use in the method according to the first aspect of the invention; and (b) detection of the presence or absence of an amplicon, wherein the presence of an amplicon is indicative for the presence of carbapenemase producing bacteria in the sample and the absence of an amplicon is indicative for the absence of carbapenemase producing bacteria in the sample.

The term "is indicative for" or "indicates" as used herein refers to an act of identifying or specifying the thing (i.e. the presence or absence of carbapenemase producing bacteria) to be indicated. As will be understood by persons skilled in the art, such assessment normally may not be correct for 100% of the patients, although it preferably is correct. The term, however, requires that a correct indication can be made for a statistically significant part of the subjects.

The term "amplification" as used herein refers to an increase in the number of copies of the target nucleic acid and its complementary sequence, or particularly a region thereof. The amplification may be performed by using any method known in the art. The amplification of nucleic acid includes methods that require multiple cycles during the amplification process or method that are performed at a single temperature. Cycling techniques are exemplified by methods requiring thermo-cycling. The methods requiring thermo-cycling include polymerase chain reaction (PCR), which is well known in the art. The PCR includes denaturing a double-stranded DNA into single stranded DNAs by thermal denaturation, annealing a primer to the single stranded DNAs; and synthesizing a complementary strand from the primer. An "amplicon" is a double-stranded fragment of DNA generated by amplification, preferably by PCR. In preferred embodiments of the method according to the first aspect of the invention, the amplicon has a size of 70-500 bp, preferably 100-400 bp, more preferably 100-300 bp, even more preferably 100-220 bp. In preferred embodiments, the distance between primers and probes is less than 100 bp, preferably less than 75 bp, preferably less than 50 bp, even more preferably less than 25 bp.

It is preferred that the amplification is by PCR, more preferably by multiplex PCR.

In the context of the present specification, the term "multiplex PCR" refers to the use of PCR to amplify several different DNA sequences simultaneously. This process amplifies DNA in samples using multiple primer pairs specific for different target nucleic acid sequences.

It is further preferred that the amplification is by quantitative PCR (qPCR), more preferably by quantitative multiplex PCR.

In the context of the present specification, the term "quantitative PCR (qPCR)" refers to a PCR in which the amplification of a target nucleic acid sequences is monitored during the PCR (i.e., in "real time"), not at its end, as in conventional PCR. Common methods for the quantitative detection of PCR products in qPCR are non-specific fluorescent dyes that intercalate with any double-stranded amplification product and sequence-specific DNA probes which permit sequence-specific detection of an amplification product after hybridization of the probe with its complementary sequence. In preferred embodiments of the method according to the first aspect of the invention, quantitative multiplex PCR with sequence specific probes is used.

The method according to the first aspect of the invention enables the simultaneous detection of multiple relevant carbapenemase genes. As used herein, the term "simultaneous" when referring to amplification and/or detection generally refers to events (e.g. amplification and/or detection) that are contemporaneous and/or synchronized in order to occur in a single tube or in the course of a single process (e.g. a PCR reaction). As such, "simultaneous" is not limited to events that occur exactly at the same time. It can be envisioned that a simultaneous detection of multiple carbapenemase genes occurs in one multiplex PCR reaction or several separate multiplex PCR reactions. In preferred embodiments, simultaneous detection of the presence or absence of at least six, preferably at least seven, more preferably at least eight carbapenemase genes is determined in one or two multiplex PCR reactions.

The use of a multiplex PCR in the method according to the first aspect of the invention, in particular a multiplex PCT with not more than two separate multiplex reactions, provides for a significant reduction in time needed for determining the presence or absence of carbapenem producing bacteria. The inventors have shown that the method according to the first aspect of the invention allows for detection of the presence or absence of eighth carbapenemase genes (including their variants) in less than 3 hours (starting from bacterial colonies).

The method according to the first aspect of the invention detects the presence or absence of additional carbapenemase genes besides a KPC carbapenemase gene, an NDM carbapenemase gene, a VIM carbapenemase gene and an OXA-48-like carbapenemase gene. Thus, in certain embodiments, the presence or absence of at least one or at least two carbapenemase genes selected from the group consisting of an IMP carbapenemase gene, an OXA-23-like carbapenemase gene, an OXA-40/24-like carbapenemase gene and an OXA-58-like carbapenemase gene is determined. Preferably, the presence or absence of at least one carbapenemase gene selected from the group consisting of an OXA-40/24-like carbapenemase gene and an OXA-58-like carbapenemase gene is determined.

In most preferred embodiments, the presence or absence of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/42-like carbapenemase gene is determined.

In the method according to the first aspect of the invention, a forward primer and the corresponding reverse primer form a primer pair. The corresponding probe (if present) is capable of hybridization with one strand of an amplicon generatable with the primer pair.

The forward primer, the corresponding reverse primer and (if present) the corresponding probe have, with respect to a double-stranded nucleic acid molecule, sequences that are (at least substantially) identical to one strand each such that they each anneal to the complementary strand of the strand they are (at least substantially) identical to. The term "forward primer" refers to the primer which is (at least substantially) identical to the forward strand (as defined by the direction of the genomic reference sequence) of the double-stranded nucleic acid molecule, and the term "reverse primer" refers to the primer which is (at least substantially) identical to the reverse complementary strand of the forward strand in the double-stranded nucleic acid molecule. The term "probe" refers to an oligonucleotide which is (at least substantially) identical to either the forward or the reverse strand of the double-stranded nucleic acid molecule. The distance between the sites where forward and reverse primer anneal to their template depends on the length of the amplicon the primers are supposed to allow generating. Typically, with respect to the present invention it is between 100 and 220 bp.

Together, a forward primer, the corresponding reverse primer and optionally the corresponding probe form an oligonucleotide set. It is preferred that the oligonucleotide set comprises a probe. As an example, F1 and R1 form a primer pair, and P1 (if present) is capable of hybridization with one strand of an amplicon generatable with F1 and R1. Together, F1, R1 and optionally P1 form oligonucleotide set 1. The oligonucleotide sets 1 to 8 are suitable for determining the presence or absence of a carbapenemase gene, wherein each oligonucleotide set allows for the detection of a certain carbapenemase gene (including its variants as described herein). For example, oligonucleotide set 1 allows for the detection of an NDM carbapenemase gene. For each oligonucleotide set, several alternatives exist.

The following embodiments relate to possible sequences for each oligonucleotide set. The primers comprise or consist of 16 to 30 consecutive nucleotides of the indicated sequences. The probes (if present) comprise or consist of 20 to 30 consecutive nucleotides of the indicated sequences. The indicated sequences were determined such that the selection of 16 to 30 (or 20 to 30, respectively) consecutive nucleotides results in primers/probes showing a selective amplification of their respective carbapenemase gene and having a low probability of hairpin formation or dimer formation (ΔG of less than -9.0 kcal/mol).

In some embodiments, the sequence of F1 comprises or consists of 16 to 30 consecutive nucleotides of SEQ ID NO:3, the sequence of R1 comprises or consists of 16 to 30 consecutive nucleotides of SEQ ID NO:4 and the sequence of P1 comprises or consists of 20 to 30 consecutive nucleotides of SEQ ID NO:5. Alternatively, the sequence of F1 comprises or consists of 16 to 30 consecutive nucleotides of SEQ ID NO:6, the sequence of R1 comprises or consists of 16 to 30 consecutive nucleotides of SEQ ID NO:7 and the sequence of P1 comprises or consists of 20 to 30 consecutive nucleotides of SEQ ID NO:8. Alternatively, the sequence of F1 comprises or consists of 16 to 30 consecutive nucleotides of SEQ ID NO:9, the sequence of R1 comprises or consists of 16 to 30 consecutive nucleotides of SEQ ID NO:10 and the sequence of P1 comprises or consists of 20 to 30 consecutive nucleotides of SEQ ID NO:11. Alternatively, the sequence of F1 comprises or consists of 16 to 30 consecutive nucleotides of SEQ ID NO:12, the sequence of R1 comprises or consists of 16 to 30 consecutive nucleotides of SEQ ID NO:13 and the sequence of P1 comprises or consists of 20 to 30 consecutive nucleotides of SEQ ID NO:14.

In some embodiments, the sequence of F2 comprises or consists of 16 to 30 consecutive nucleotides of SEQ ID NO:29, the sequence of R2 comprises or consists of 16 to 30 consecutive nucleotides of SEQ ID NO:30 and the sequence of P2 comprises or consists of 20 to 30 consecutive nucleotides of SEQ ID NO:31. Alternatively, the sequence of F2 comprises or consists of 16 to 30 consecutive nucleotides of SEQ ID NO:32, the sequence of R2 comprises or consists of 16 to 30 consecutive nucleotides of SEQ ID NO:33 and the sequence of P2 comprises or consists of 20 to 30 consecutive nucleotides of SEQ ID NO:34. Alternatively, the sequence of F2 comprises or consists of 16 to 30 consecutive nucleotides of SEQ ID NO:35, the sequence of R2 comprises or consists of 16 to 30 consecutive nucleotides of SEQ ID NO:36 and the sequence of P2 comprises or consists of 20 to 30 consecutive nucleotides of SEQ ID NO:37.

In some embodiments, the sequence of F3 comprises or consists of 16 to 30 consecutive nucleotides of SEQ ID NO:49, the sequence of R3 comprises or consists of 16 to 30 consecutive nucleotides of SEQ ID NO:50 and the sequence of P3 comprises or consists of 20 to 30 consecutive nucleotides of SEQ ID NO:51. Alternatively, the sequence of F3 comprises or consists of 16 to 30 consecutive nucleotides of SEQ ID NO:52, the sequence of R3 comprises or consists of 16 to 30 consecutive nucleotides of SEQ ID NO:53 and the sequence of P3 comprises or consists of 20 to 30 consecutive nucleotides of SEQ ID NO:54. Alternatively, the sequence of F3 comprises or consists of 16 to 30 consecutive nucleotides of SEQ ID NO:55, the sequence of R3 comprises or consists of 16 to 30 consecutive nucleotides of SEQ ID NO:56 or SEQ ID NO:57 and the sequence of P3 comprises or consists of 20 to 30 consecutive nucleotides of SEQ ID NO:58.

In some embodiments, the sequence of F4 comprises or consists of 16 to 30 consecutive nucleotides of SEQ ID NO:70, the sequence of R4 comprises or consists of 16 to 30 consecutive nucleotides of SEQ ID NO:71 and the sequence of P4 comprises or consists of 20 to 30 consecutive nucleotides of SEQ ID NO:72. Alternatively, the sequence of F4 comprises or consists of 16 to 30 consecutive nucleotides of SEQ ID NO:80, the sequence of R4 comprises or consists of 16 to 30 consecutive nucleotides of SEQ ID NO:81 and the sequence of P4 comprises or consists of 20 to 30 consecutive nucleotides of SEQ ID NO:82. Alternatively, the sequence of F4 comprises or consists of 16 to 30 consecutive nucleotides of SEQ ID NO:87, the sequence of R4 comprises or consists of 16 to 30 consecutive nucleotides of SEQ ID NO:88 and the sequence of P4 comprises or consists of 20 to 30 consecutive nucleotides of SEQ ID NO:89.

In some embodiments, the sequence of F5 comprises or consists of 16 to 30 consecutive nucleotides of SEQ ID NO:95, the sequence of R5 comprises or consists of 16 to 30 consecutive nucleotides of SEQ ID NO:96 and the sequence of P5 comprises or consists of 20 to 30 consecutive nucleotides of SEQ ID NO:97. Alternatively, the sequence of F5 comprises or consists of 16 to 30 consecutive nucleotides of SEQ ID NO:98, the sequence of R5 comprises or consists of 16 to 30 consecutive nucleotides of SEQ ID NO:99 and the sequence of P5 comprises or consists of 20 to 30 consecutive nucleotides of SEQ ID NO:100. Alternatively, the sequence of F5 comprises or consists of 16 to 30 consecutive nucleotides of SEQ ID NO:101, the sequence of R5 comprises or consists of 16 to 30 consecutive nucleotides of SEQ ID NO:102 and the sequence of P5 comprises or consists of 20 to 30 consecutive nucleotides of SEQ ID NO:103.

In some embodiments, the sequence of F6 comprises or consists of 16 to 30 consecutive nucleotides of SEQ ID NO:115, the sequence of R6 comprises or consists of 16 to 30 consecutive nucleotides of SEQ ID NO:116 and the sequence of P6 comprises or consists of 20 to 30 consecutive nucleotides of SEQ ID NO:117. Alternatively, the sequence of F6 comprises or consists of 16 to 30 consecutive nucleotides of SEQ ID NO:118, the sequence of R6 comprises or consists of 16 to 30 consecutive nucleotides of SEQ ID NO:119 and the sequence of P6 comprises or consists of 20 to 30 consecutive nucleotides of SEQ ID NO:120.

In some embodiments, the sequence of F7 comprises or consists of 16 to 30 consecutive nucleotides of SEQ ID NO:128 or SEQ ID NO:131, the sequence of R7 comprises or consists of 16 to 30 consecutive nucleotides of SEQ ID NO:129 and the sequence of P7 comprises or consists of 20 to 30 consecutive nucleotides of SEQ ID NO:130 or SEQ ID NO:132.

In some embodiments, the sequence of F8 comprises or consists of 16 to 30 consecutive nucleotides of SEQ ID NO:139, the sequence of R8 comprises or consists of 16 to 30 consecutive nucleotides of SEQ ID NO:140 and the sequence of P8 comprises or consists of 20 to 30 consecutive nucleotides of SEQ ID NO:141 or SEQ ID NO:142.

In preferred embodiments of oligonucleotide set 1, the sequence of F1 comprises or consists of SEQ ID NO:15, the sequence of R1 comprises or consists of SEQ ID NO:16 and the sequence of P1 comprises or consists of SEQ ID NO:17; or the sequence of F1 comprises or consists of SEQ ID NO:18, the sequence of R1 comprises or consists of SEQ ID NO:19 and the sequence of P1 comprises or consists of SEQ ID NO:20; or the sequence of F1 comprises or consists of SEQ ID NO:21, the sequence of R1 comprises or consists of SEQ ID NO:22 and the sequence of P1 comprises or consists of SEQ ID NO:23; or the sequence of F1 comprises or consists of SEQ ID NO:24, the sequence of R1 comprises or consists of SEQ ID NO:25 and the sequence of P1 comprises or consists of SEQ ID NO:26.

In preferred embodiments of oligonucleotide set 2, the sequence of F2 comprises or consists of SEQ ID NO:38, the sequence of R2 comprises or consists of SEQ ID NO:39 and the sequence of P2 comprises or consists of SEQ ID NO:40; or the sequence of F2 comprises or consists of SEQ ID NO:41, the sequence of R2 comprises or consists of SEQ ID NO:42 and the sequence of P2 comprises or consists of SEQ ID NO:43; or the sequence of F2 comprises or consists of SEQ ID NO:44, the sequence of R2 comprises or consists of SEQ ID NO:45 and the sequence of P2 comprises or consists of SEQ ID NO:46.

In preferred embodiments of oligonucleotide set 3, the sequence of F3 comprises or consists of SEQ ID NO:59, the sequence of R3 comprises or consists of SEQ ID NO:60 and the sequence of P3 comprises or consists of SEQ ID NO:61; or the sequence of F3 comprises or consists of SEQ ID NO:62, the sequence of R3 comprises or consists of SEQ ID NO:63 and the sequence of P3 comprises or consists of SEQ ID NO:64; or the sequence of F3 comprises or consists of SEQ ID NO:65, the sequence of R3 comprises or consists of SEQ ID NO:66 or SEQ ID NO:67 and the sequence of P3 comprises or consists of SEQ ID NO:68.

In preferred embodiments of oligonucleotide set 4, the sequence of F4 comprises or consists of SEQ ID NO:73, the sequence of R4 comprises or consists of SEQ ID NO:74 and the sequence of P4 comprises or consists of SEQ ID NO:75; or the sequence of F4 comprises or consists of SEQ ID NO:76, the sequence of R4 comprises or consists of SEQ ID NO:77 and the sequence of P4 comprises or consists of SEQ ID NO:78; or the sequence of F4 comprises or consists of SEQ ID NO:83, the sequence of R4 comprises or consists of SEQ ID NO:84 and the sequence of P4 comprises or consists of SEQ ID NO:85; or the sequence of F4 comprises or consists of SEQ ID NO:90, the sequence of R4 comprises or consists of SEQ ID NO:91 and the sequence of P4 comprises or consists of SEQ ID NO:92.

In preferred embodiments of oligonucleotide set 5, the sequence of F5 comprises or consists of SEQ ID NO:104, the sequence of R5 comprises or consists of SEQ ID NO:105 and the sequence of P5 comprises or consists of SEQ ID NO:106; or the sequence of F5 comprises or consists of SEQ ID NO:107, the sequence of R5 comprises or consists of SEQ ID NO:108 and the sequence of P5 comprises or consists of SEQ ID NO:109; or the sequence of F5 comprises or consists of SEQ ID NO:110, the sequence of R5 comprises or consists of SEQ ID NO:111 and the sequence of P5 comprises or consists of SEQ ID NO:112.

In preferred embodiments of oligonucleotide set 6, the sequence of F6 comprises or consists of SEQ ID NO:121, the sequence of R6 comprises or consists of SEQ ID NO:122 and the sequence of P6 comprises or consists of SEQ ID NO:123; or the sequence of F6 comprises or consists of SEQ ID NO:124, the sequence of R6 comprises or consists of SEQ ID NO:125 and the sequence of P6 comprises or consists of SEQ ID NO:126.

In preferred embodiments of oligonucleotide set 7, the sequence of F7 comprises or consists of SEQ ID NO:133 or SEQ ID NO:136, the sequence of R7 comprises or consists of SEQ ID NO:134 and the sequence of P7 comprises or consists of SEQ ID NO:135 or SEQ ID NO:137.

In preferred embodiments of oligonucleotide set 8, the sequence of F8 comprises or consists of SEQ ID NO:143, the sequence of R8 comprises or consists of SEQ ID NO:144 and the sequence of P8 comprises or consists of SEQ ID NO:145 or SEQ ID NO:146.

While several alternatives exist for each oligonucleotide set, the inventors have determined a preferred set for each oligonucleotide set. The following embodiments relate to the preferred oligonucleotide sets.

In preferred embodiments of the method according to the first aspect of the invention,
a) F1, R1 and P1 are each capable of hybridization to SEQ ID NO:2 or its complement;
b) F2, R2 and P2 are each capable of hybridization to SEQ ID NO:28 or its complement;
c) F3, R3 and P3 are each capable of hybridization to SEQ ID NO:48 or its complement;
d) F4, R4 and P4 are each capable of hybridization to SEQ ID NO:69 or its complement;
e) F5, R5 and P5 are each capable of hybridization to SEQ ID NO:94 or its complement;
f) F6, R6 and P6 are each capable of hybridization to SEQ ID NO:114 or its complement;
g) F7, R7 and P7 are each capable of hybridization to SEQ ID NO:127 or its complement; and/or
h) F8, R8 and P8 are each capable of hybridization to SEQ ID NO:138 or its complement.

In more preferred embodiments of the method according to the first aspect of the invention, the primers comprise or consist of 16 to 30 consecutive nucleotides of SEQ ID NO: 3, 4, 29, 30, 49, 50, 70, 71, 95, 96, 115, 116, 128, 129, 139 or 140 and the probes (if present) comprise or consist of 20 to 30 consecutive nucleotides of SEQ ID NO:5, 31, 51, 72, 97, 117, 130 or 141. In these embodiments,
a) the sequence of F1 comprises or consists of 16 to 30, preferably approximately 20 consecutive nucleotides of SEQ ID NO:3, the sequence of R1 comprises or consists of 16 to 30, preferably approximately 17 consecutive nucleotides of SEQ ID NO:4 and the sequence of P1 comprises or consists of 20 to 30, preferably approximately 24 consecutive nucleotides of SEQ ID NO:5;
b) the sequence of F2 comprises or consists of 16 to 30, preferably approximately 18 consecutive nucleotides of SEQ ID NO:29, the sequence of R2 comprises or consists of 16 to 30, preferably approximately 18 consecutive nucleotides of SEQ ID NO:30 and the sequence of P2 comprises or consists of 20 to 30, preferably approximately 21 consecutive nucleotides of SEQ ID NO:31;
c) the sequence of F3 comprises or consists of 16 to 30, preferably approximately 19 consecutive nucleotides of SEQ ID NO:49, the sequence of R3 comprises or consists of 16 to 30, preferably approximately 18 consecutive nucleotides of SEQ ID NO:50 and the sequence of P3 comprises or consists of 20 to 30, preferably approximately 29 consecutive nucleotides of SEQ ID NO:51;
d) the sequence of F4 comprises or consists of 16 to 30, preferably approximately 22 consecutive nucleotides of SEQ ID NO:70, the sequence of R4 comprises or consists of 16 to 30, preferably approximately 21 consecutive nucleotides of SEQ ID NO:71 and the sequence of P4 comprises or consists of 20 to 30, preferably approximately 28 consecutive nucleotides of SEQ ID NO:72;
e) the sequence of F5 comprises or consists of 16 to 30, preferably approximately 18 consecutive nucleotides of SEQ ID NO:95, the sequence of R5 comprises or consists of 16 to 30, preferably approximately 19 consecutive nucleotides of SEQ ID NO:96 and the sequence of P5 comprises or consists of 20 to 30, preferably approximately 26 consecutive nucleotides of SEQ ID NO:97;
f) the sequence of F6 comprises or consists of 16 to 30, preferably approximately 17 consecutive nucleotides of SEQ ID NO:115, the sequence of R6 comprises or consists of 16 to 30, preferably approximately 20 consecutive nucleotides of SEQ ID NO:116 and the sequence of P6 comprises or consists of 20 to 30, preferably approximately 23 consecutive nucleotides of SEQ ID NO:117;
g) the sequence of F7 comprises or consists of 16 to 30, preferably approximately 18 consecutive nucleotides of SEQ ID NO:128, the sequence of R7 comprises or consists of 16 to 30, preferably approximately 19 consecutive nucleotides of SEQ ID NO:129 and the sequence of P7 comprises or consists of 20 to 30, preferably approximately 28 consecutive nucleotides of SEQ ID NO:130; and/or
h) the sequence of F8 comprises or consists of 16 to 30, preferably approximately 19 consecutive nucleotides of SEQ ID NO:139, the sequence of R8 comprises or consists of 16 to 30, preferably approximately 20 consecutive nucleotides of SEQ ID NO:140 and the sequence of P8 comprises or consists of 20 to 30, preferably approximately 27 consecutive nucleotides of SEQ ID NO:141.

In even more preferred embodiments of the method according to the first aspect of the invention,
a) the sequence of F1 comprises or consists of SEQ ID NO:15, the sequence of R1 comprises or consists of SEQ ID NO:16 and the sequence of P1 comprises or consists of SEQ ID NO:17;
b) the sequence of F2 comprises or consists of SEQ ID NO:38, the sequence of R2 comprises or consists of SEQ ID NO:39 and the sequence of P2 comprises or consists of SEQ ID NO:40;
c) the sequence of F3 comprises or consists of SEQ ID NO:59, the sequence of R3 comprises or consists of SEQ ID NO:60 and the sequence of P3 comprises or consists of SEQ ID NO:61;
d) the sequence of F4 comprises or consists of SEQ ID NO:73, the sequence of R4 comprises or consists of SEQ ID NO:74 and the sequence of P4 comprises or consists of SEQ ID NO:75;
e) the sequence of F5 comprises or consists of SEQ ID NO:104, the sequence of R5 comprises or consists of SEQ ID NO:105 and the sequence of P5 comprises or consists of SEQ ID NO:106;
f) the sequence of F6 comprises or consists of SEQ ID NO:121, the sequence of R6 comprises or consists of SEQ ID NO:122 and the sequence of P6 comprises or consists of SEQ ID NO:123;
g) the sequence of F7 comprises or consists of SEQ ID NO:133, the sequence of R7 comprises or consists of SEQ ID NO:134 and the sequence of P7 comprises or consists of SEQ ID NO:135; and/or
h) the sequence of F8 comprises or consists of SEQ ID NO:143, the sequence of R8 comprises or consists of SEQ ID NO:144 and the sequence of P8 comprises or consists of SEQ ID NO:145.

In preferred embodiments, the presence of the IMP carbapenemase gene is determined using a forward primer F4 and a forward primer F4.2, a reverse primer R4 and a reverse primer R4.2 and optionally a probe P4 and a probe P4.2, wherein F4, F4.2, R4, R4.2, P4 and P4.2 are each capable of hybridization to SEQ ID NO:69 or its complement. In other words, in preferred embodiments, the presence of the IMP carbapenemase gene is determined using two oligonucleotide sets (set 4 and set 4.2), wherein these sets are different from each other and allow for the detection of additional IMP carbapenemase genes. The oligonucleotides of set 4.2 are elongated with respect to the oligonucleotides of set 4. The elongation serves to stabilize mismatches between the oligonucleotides and some IMP carbapenemase genes. In some embodiments of oligonucleotide set 4.2, the sequence of F4.2 comprises or consists of 20 to 30, preferably approximately 25 consecutive nucleotides of SEQ ID NO:80, the sequence of R4.2 comprises or consists of 20 to 30, preferably approximately 24 consecutive nucleotides of SEQ ID NO:81 and the sequence of P4.2 comprises or consists of 25 to 40, preferably approximately 35 consecutive nucleotides of SEQ ID NO:82. In preferred embodiments of oligonucleotide set 4.2, the sequence of F4.2 comprises or consists of SEQ ID NO:76, the sequence of R4.2 comprises or consists of SEQ ID NO:77 and the sequence of P4.2 comprises or consists of SEQ ID NO:78.

In most preferred embodiments of the method according to the first aspect of the invention,
a) the sequence of F1 comprises or consists of SEQ ID NO: 15, the sequence of R1 comprises or consists of SEQ ID NO:16 and the sequence of P1 comprises or consists of SEQ ID NO: 17;
b) the sequence of F2 comprises or consists of SEQ ID NO:38, the sequence of R2 comprises or consists of SEQ ID NO:39 and the sequence of P2 comprises or consists of SEQ ID NO:40;
c) the sequence of F3 comprises or consists of SEQ ID NO:59, the sequence of R3 comprises or consists of SEQ ID NO:60 and the sequence of P3 comprises or consists of SEQ ID NO:61;
d) the sequence of F4 comprises or consists of SEQ ID NO:73, the sequence of R4 comprises or consists of SEQ ID NO:74 and the sequence of P4 comprises or consists of SEQ ID NO:75 and the sequence of F4.2 comprises or consists of SEQ ID NO:76, the sequence of R4.2 comprises or consists of SEQ ID NO:77 and the sequence of P4.2 comprises or consists of SEQ ID NO:78;
e) the sequence of F5 comprises or consists of SEQ ID NO: 104, the sequence of R5 comprises or consists of SEQ ID NO: 105 and the sequence of P5 comprises or consists of SEQ ID NO: 106;
f) the sequence of F6 comprises or consists of SEQ ID NO: 121, the sequence of R6 comprises or consists of SEQ ID NO: 122 and the sequence of P6 comprises or consists of SEQ ID NO: 123;
g) the sequence of F7 comprises or consists of SEQ ID NO: 133, the sequence of R7 comprises or consists of SEQ ID NO: 134 and the sequence of P7 comprises or consists of SEQ ID NO: 135; and/or
h) the sequence of F8 comprises or consists of SEQ ID NO: 143, the sequence of R8 comprises or consists of SEQ ID NO: 144 and the sequence of P8 comprises or consists of SEQ ID NO: 145.

The method according to the first aspect of the invention can easily be automatized, thereby allowing an easy performance of DNA extraction and real-time PCR without the need of increased experience of the person performing the assay.

In a second aspect, the present invention relates to an oligonucleotide set comprising
a) a forward primer F1 comprising or consisting of SEQ ID NO: 15, a reverse primer R1 comprising or consisting of SEQ ID NO:16 and a probe P1 comprising or consisting of SEQ ID NO: 17;
b) a forward primer F2 comprising or consisting of SEQ ID NO:38, a reverse primer R2 comprising or consisting of SEQ ID NO:39 and a probe P2 comprising or consisting of SEQ ID NO:40;
c) a forward primer F3 comprising or consisting of SEQ ID NO:59, a reverse primer R3 comprising or consisting of SEQ ID NO:60 and a probe P3 comprising or consisting of SEQ ID NO:61;
d) a forward primer F4 comprising or consisting of SEQ ID NO:73, a reverse primer R4 comprising or consisting of SEQ ID NO:74 and a probe P4 comprising or consisting of SEQ ID NO:75;
e) a forward primer F5 comprising or consisting of SEQ ID NO: 104, a reverse primer R5 comprising or consisting of SEQ ID NO: 105 and a probe P5 comprising or consisting of SEQ ID NO: 106;
f) a forward primer F6 comprising or consisting of SEQ ID NO: 121, a reverse primer R6 comprising or consisting of SEQ ID NO: 122 and a probe P6 comprising or consisting of SEQ ID NO: 123;
g) a forward primer F7 comprising or consisting of SEQ ID NO: 133, a reverse primer R7 comprising or consisting of SEQ ID NO: 134 and a probe P7 comprising or consisting of SEQ ID NO: 135; and/or
h) a forward primer F8 comprising or consisting of SEQ ID NO: 143, a reverse primer R8 comprising or consisting of SEQ ID NO: 144 and a probe P8 comprising or consisting of SEQ ID NO: 145.

In preferred embodiments of the second aspect of the invention, each of the oligonucleotide sets a to h comprises a forward primer, a reverse primer and a probe. It is preferred that in addition, the oligonucleotide set comprises a forward primer F4.2 comprising or consisting of SEQ ID NO:76, a reverse primer R4.2 comprising or consisting of SEQ ID NO:77 and a probe P4.2 comprising or consisting of SEQ ID NO:78.

In a third aspect, the present invention relates to a kit comprising an oligonucleotide set according to the second aspect of the invention and a component selected from the group consisting of a buffer, nucleotides and a thermostable DNA polymerase.

Oligonucleotides of the invention (e.g. primers and probes) may be prepared using general methods well known in the art, such as synthesis from appropriate nucleotide triphosphates, isolation from biological sources, etc. Synthetic oligonucleotides may be obtained using nucleic acid synthesizers or similar devices. The resultant construct may be purified according to methods known in the art, such as high-performance liquid chromatography (HPLC). Long, double-stranded polynucleotides may be synthesized in stages, due to any size limitations inherent in the oligonucleotide synthesis methods.

In a fourth aspect, the present invention relates to a use of the oligonucleotide set according to second aspect or the kit according the third aspect for determining in a sample the presence or absence of carbapenemase producing bacteria.

In a fifth aspect, the present invention relates to a method of diagnosis of an infection with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene; wherein the presence of a carbapenemase gene in the sample is indicative of an infection with carbapenemase producing bacteria.

In preferred embodiments, the method of diagnosis is an in vitro method.

The method of diagnosis according to the fifth aspect includes the detection of the onset of an infection with carbapenemase producing bacteria, i.e. before an overtly observable clinical manifestation. The method of diagnosis according to the fifth aspect may be used as an initial screening to identify patients colonized with carbapenemase harboring bacteria. The reduced time until results are obtained can avoid isolation of patients and will help to prevent spread of antimicrobial resistances and possible outbreaks by timely initiation of infection control measures.

In a sixth aspect, the present invention relates to a method of treatment of a bacterial infection, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample comprises carbapenemase producing bacteria, administering to the patient an antibacterial drug different from a beta lactam antibacterial drug, in particular different from a carbapenem, preferably an antibacterial drug selected from the group consisting of colistin, gentamicin, ceftazidime-avibactam, ceftolozane-tazobactam, fosfomycin, nitrofurantoin, pivmecillinam and temocillin; if the sample does not comprise carbapenemase producing bacteria, administering to the patient beta lactam antibacterial drug, preferably a carbapenem.

The terms "treatment" or "treating" as used herein refer to a therapeutic treatment, wherein the goal is to cure a bacterial infection.

In a seventh aspect, the present invention relates to an in vitro method for selecting a patient for treatment with a beta lactam antibacterial drug, preferably a carbapenem, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, selecting the patient for treatment with a beta lactam antibacterial drug, preferably a carbapenem.

In an eighth aspect, the present invention relates to a beta lactam antibacterial drug, preferably a carbapenem for use in a method of treatment of a bacterial infection in a patient that is not infected with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, administering to the patient a beta lactam antibacterial drug, preferably a carbapenem.

In a further aspect, the present invention relates to the oligonucleotide set according to the second aspect or the kit according to the third aspect for use in a method of treatment of a bacterial infection in a patient that is not infected with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, administering to the patient a beta lactam antibacterial drug, preferably a carbapenem.

In preferred embodiments of the method of diagnosis according to the fifth aspect, the method of treatment according to the sixth aspect, the in vitro method according to the seventh aspect or the beta lactam antibacterial drug, preferably a carbapenem, for use according to the eighth aspect or the oligonucleotide set or the kit for use according to the further aspect, step b) comprises determining the presence or absence of carbapenemase producing bacteria according to the method of the first aspect of the invention.

The probes, primers, kits and methods described herein may also be useful in implementing adequate hygiene procedures, for instance by providing detection of presence or absences of bacteria in various environments including, but not limited to, hospitals (e.g. laboratory working surfaces, medical instruments, patient rooms, etc.), public spaces (e.g. schools, shopping malls, etc.), nature (e.g. water, air, soil, etc.) and the like. The invention may also find applications in epidemiologic studies.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

The present application refers to SEQ ID NOs 1-149. An overview and explanation of these SEQ ID NOs is given in Table 1 below:

**Table 1: Sequences**

| Gene | | | | SEQ ID NO |
|---|---|---|---|---|
| NDM | Consensus | | | 1 |
| | Consensus short | | | 2 |
| | Primer & Probe Regions | Set 1.1 | F | 3 |
| | | | R | 4 |
| | | | P | 5 |
| | | Set 1.2 | F | 6 |
| | | | R | 7 |
| | | | P | 8 |
| | | Set 1.3 | F | 9 |
| | | | R | 10 |
| | | | P | 11 |
| | | Set 1.4 | F | 12 |
| | | | R | 13 |
| | | | P | 14 |
| | Primer & Probe | Set 1.1 | F | 15 |
| | | | R | 16 |
| | | | P | 17 |
| | | Set 1.2 | F | 18 |
| | | | R | 19 |
| | | | P | 20 |
| | | Set 1.3 | F | 21 |
| | | | R | 22 |
| | | | P | 23 |
| | | Set 1.4 | F | 24 |
| | | | R | 25 |
| | | | P | 26 |
| KPC | Consensus | | | 27 |
| | Consensus short | | | 28 |
| | Primer & Probe Regions | Set 2.1 | F | 29 |
| | | | R | 30 |
| | | | P | 31 |
| | | Set 2.2 | F | 32 |
| | | | R | 33 |
| | | | P | 43 |
| | | Set 2.3 | F | 35 |
| | | | R | 36 |
| | | | P | 37 |
| | Primer & Probe | Set 2.1 | F | 38 |
| | | | R | 39 |
| | | | P | 40 |
| | | Set 2.2 | F | 41 |
| | | | R | 42 |
| | | | P | 43 |
| | | Set 2.3 | F | 44 |
| | | | R | 45 |
| | | | P | 46 |
| VIM | Consensus | | | 47 |
| | Consensus short | | | 48 |
| | Primer & Probe Regions | Set 3.1 | F | 49 |
| | | | R | 50 |
| | | | P | 51 |
| | | Set 3.2 | F | 52 |
| | | | R | 53 |
| | | | P | 54 |
| | | Set 3.3 | F | 55 |
| | | | R | 56 |
| | | | R2 | 57 |
| | | | P | 58 |
| | Primer & Probe | Set 3.1 | F | 59 |
| | | | R | 60 |
| | | | P | 61 |
| | | Set 3.2 | F | 62 |
| | | | R | 63 |
| | | | P | 64 |
| | | Set 3.3 | F | 65 |
| | | | R | 66 |
| | | | R2 | 67 |
| | | | P | 68 |
| IMP | Consensus 1 | | | 69 |
| | Primer & Probe Regions | Set 4.1&2 | F | 70 |
| | | | R | 71 |
| | | | P | 72 |
| | Primer & Probe | Set 4.1 | F | 73 |
| | | | R | 74 |
| | | | P | 75 |
| | | Set 4.2 | F | 76 |
| | | | R | 77 |
| | | | P | 78 |
| | Consensus 2 | | | 79 |
| | Primer & Probe Regions | Set 4.3 | F | 80 |
| | | | R | 81 |
| | | | P | 82 |
| | Primer & Probe | Set 4.3 | F | 83 |
| | | | R | 84 |
| | | | P | 85 |
| | Consensus 3 | | | 86 |
| | Primer & Probe Regions | Set 4.3 | F | 87 |
| | | | R | 88 |
| | | | P | 89 |
| | Primer & Probe | Set 4.3 | F | 90 |
| | | | R | 91 |
| | | | P | 92 |
| OXA-23-like | Consensus | | | 93 |
| | Consensus short | | | 94 |
| | Primer & Probe Regions | Set 5.1 | F | 95 |
| | | | R | 96 |
| | | | P | 97 |
| | | Set 5.2 | F | 98 |
| | | | R | 99 |
| | | | P | 100 |
| | | Set 5.3 | F | 101 |
| | | | R | 102 |
| | | | P | 103 |
| | Primer & Probe | Set 5.1 | F | 104 |
| | | | R | 105 |
| | | | P | 106 |
| | | Set 5.2 | F | 107 |
| | | | R | 108 |
| | | | P | 109 |
| | | Set 5.3 | F | 110 |
| | | | R | 111 |
| | | | P | 112 |
| OXA-48-like | Consensus | | | 113 |
| | Consensus short | | | 114 |
| | Primer & Probe Regions | Set 6.1 | F | 115 |
| | | | R | 116 |
| | | | P | 117 |
| | | Set 6.2 | F | 118 |
| | | | R | 119 |
| | | | P | 120 |
| | Primer & Probe | Set 6.1 | F | 121 |
| | | | R | 122 |
| | | | P | 123 |
| | | Set 6.2 | F | 124 |
| | | | R | 125 |
| | | | P | 126 |
| OXA-58-like | Consensus | | | 127 |
| | Primer & Probe Regions | Set 7.1 | F | 128 |
| | | | R | 129 |
| | | | P | 130 |
| | | Set 7.2 | F | 131 |
| | | | P | 132 |
| | Primer & Probe | Set 7.1 | F | 133 |
| | | | R | 134 |
| | | | P | 135 |
| | | Set 7.2 | F | 136 |
| | | | P | 137 |
| OXA-40/24-like | Consensus | | | 138 |
| | Primer & Probe Regions | Set 8.1 | F | 139 |
| | | | R | 140 |
| | | | P | 141 |
| | | Set 8.2 | P | 142 |
| | Primer & Probe | Set 8.1 | F | 143 |
| | | | R | 144 |
| | | | P | 145 |
| | | Set 8.2 | P | 146 |
| S16 | Primer & Probe | | F | 147 |
| | | | R | 148 |
| | | | P | 149 |

The invention is described by way of the following figures and examples which are to be construed as merely illustrative and not limitative of the scope of the invention.

### LEGENDS TO THE FIGURES

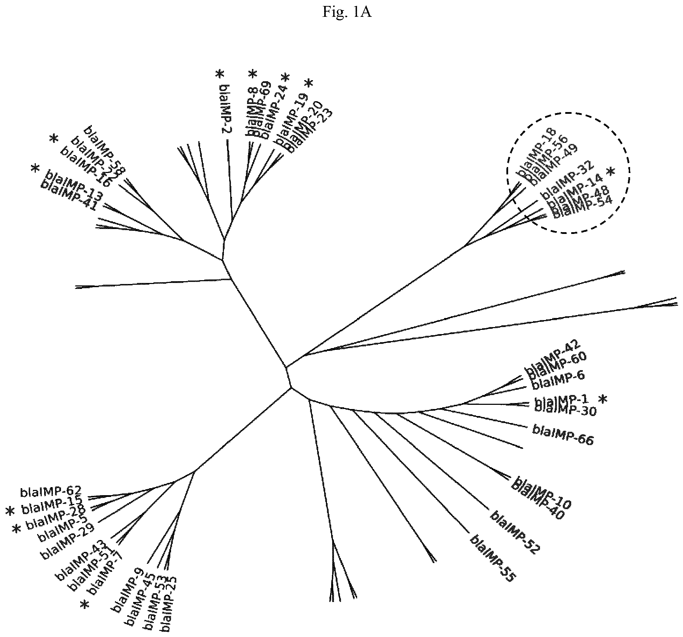

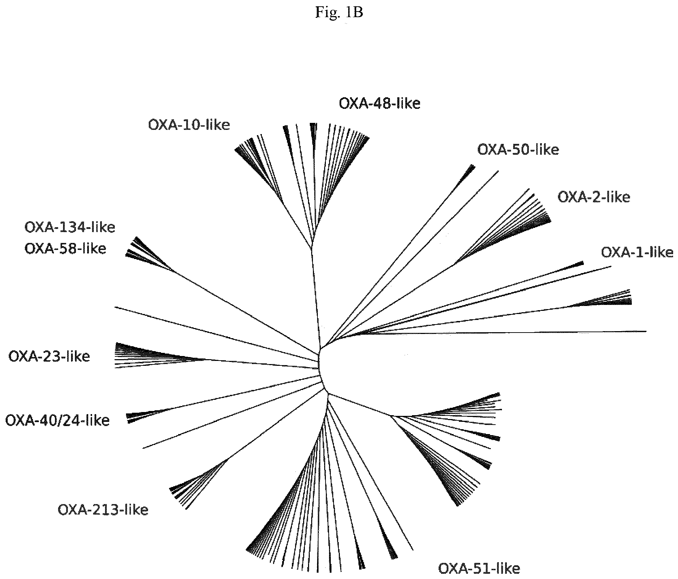

**Figure 1****: UPGMA nucleotide sequence trees of all currently known blaIMP (A) and blaOXA (B) beta-lactamases.** A: grey and circled: detected variants with primer set 2, black: variants that are detectable with either both sets or a combination of set 1 and set 2, *: experimentally tested variants; branches without label: blaIMP variants that cannot be detected with the two primer sets. B: colored tip labels are detectable with the multiplex PCR. Black tip labels represent other known blaOXA-groups with different spectrum of activity. Unlabeled branches: unclassified blaOXA variants.

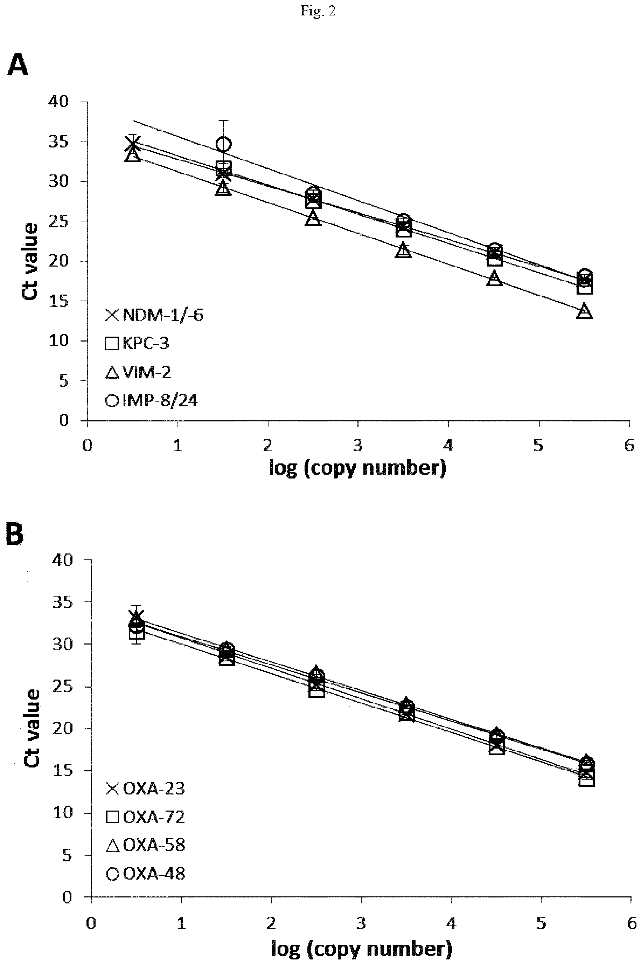

**Figure 2****: Determination of limit of detection and PCR efficiencies.** A: standard curve for MM1; the detection limit for NDM and VIM is 3.2 copies, for KPC and IMP 32 copies. PCR efficiency in MM1 is 98 % for NDM, 88 % for KPC, 80 % for VIM and 77 % for IMP. B: standard curve for MM2; the detection limit for the different OXA variants is 3.2 copies. PCR efficiencies are between 89 % and 99 % in MM2.

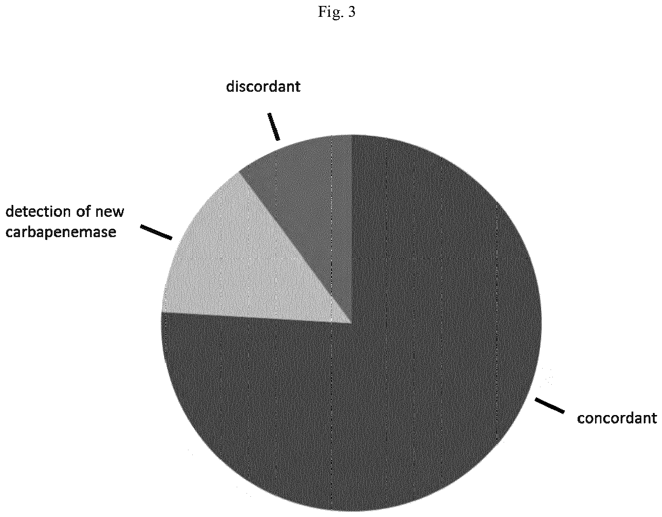

**Figure 3****: Carbapenemase detection directly from rectal swabs compared to differentiated isolates.** 60 of 79 carbapenemase genes found in the rectal swabs were also present in the differentiated isolates. 11 of 79 additional carbapenemase genes were detected by testing directly from rectal swabs. 8 of 79 carbapenemases could not be identified in the direct PCR-sample but were detected in isolates from selective agar.

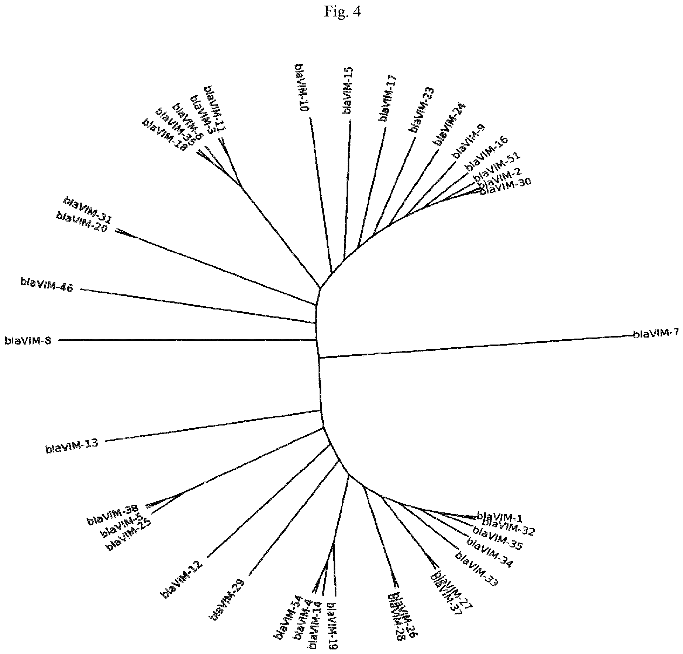

**Figure 4****: UPGMA nucleotide sequence trees of all currently known blaVIM variants.** blaVIM-7 was excluded from primer design due to high sequence variations.

### EXAMPLES

Since the primers are specific for the respective carbapenemase even without a probe, they can also be used for a classic PCR with subsequent capillary electrophoresis. The carbapenemases can be differentiated on the basis of different amplicon sizes. For KPC, VIM and OXA-48-like, an adjustment can be made to increase the size difference (see Table 3).

### Materials and Methods

### Assay design

A sequence collection of beta lactamases from the center for genomic epidemiology (https://cge.cbs.dtu.dk/services/data.php, December 2017) was used to design primers and hydrolysis probes. It was complemented with all currently available carbapenemase gene variants from the NCBI database (December 2017, Table 2). The assay was designed to detect eight common carbapenemase groups: blaNDM, blaKPC, blaVIM, blaIMP, blaOXA-23-like, blaOXA-40/24-like, blaOXA-58-like and blaOXA-48-like.

**Table 2: Sequences from NCBI added to the sequence collection from the center for genomic epidemiology**

| Carbapenemase | Accession number |
|---|---|
| NDM | NG_049332.1, NG_049333.1, KX812714.1, KY503030.1, MF370080.1, MG183694.1 |
| KPC | NG_049249.1, NG_049250.1, NG_049251.1, NG_049252.1, MF772496.1, NG_049254.1, NG_049255.1, NG_051167.1, NG_051469.1, NG_052862.1, KY282958.1, KY563764.1, KY646302.1, NG_055494.1, NG_055495.1 |
| VIM | NG_050366.1, HG934765.1, KP771862.1, NG_050370.1, NG_050371.1, NG_050372.1, NG_050373.1, NG_050374.1, KT954134.1, KT964061.1, KU663374.1, KU663375.1, NG_050379.1, KX349731.1, KY508061.1 |
| IMP | NG_049209.1, NG_049210.1, NG_049211.1, NG_049213.1, NG_049214.1, NG_049215.1, KU052795.1, KU299753.1, KU315553.1, KU647281.1, KX196782.1, NG_050945.1, KX462700.1, KX753224.1, KX821663.1, NG_052050.1, LC190726.1, MF281100.1, MF669572.1, MF678349.1 |
| OXA | KF900153.1, KU726870.1, KU878974.1, KX349732.1, KX827284.1, KX828713.1, KY094077.1, KY126221.1, KY126224.1, KY126231.1, KY126232.1, KY126233.1, |
| | KY126234.1, KY126237.1, KY126238.1, KY126240.1, KY126241.1, KY674541.1, KY682750.1, KY682751.1, KY682752.1, KY682753.1, KY682754.1, KY682755.1, KY682756.1, KY684124.1, KY767021.1, KY767022.1, KY883665.1, MF099636.1, NG_049787.1, NG_049791.1, NG_051168.1, NG_051751.1, NG_051752.1, NG_052051.1, NG_052064.1, NG_052174.1, NG_052519.1, NG_054666.1, NG_054692.1, NG_054693.1, NG_054698.1, NG_054699.1, NG_054700.1, NG_054701.1, NG_054702.1, NG_054703.1, NG_054704.1, NG_054705.1, NG_054706.1, NG_054707.1, NG_054708.1, NG_054712.1, NG_054953.1, NG_054954.1, NG_054955.1, NG_054956.1, NG_054957.1, NG_054958.1, NG_054959.1, NG_055475.1, NG_055476.1, NG_055490.1, NG_055499.1 |

The alignment of the carbapenemase sequences was done with MUSCLE in MEGA 7.0.26. The aim was to cover as many gene variants as possible with one set of primers and probe. To analyze variants clustering together, phylogenetic trees were created with the UPGMA method in MEGA 7.0.26. Either all sequences or a selection of sequences was used to create a consensus sequence. The primer and probe design was done with the IDT primer tool (Integrated DNA Technologies, Inc.), with following settings: Mg²⁺: 4 mM, Tm primer: 58 °C - 61 °C, Tm probe: 68 °C - 72 °C, amplicon size: 100 bp - 220 bp.

The given primers and probes were analyzed with the IDT oligo analyzer for hairpin formation and self-/heterodimers, regarding the IDT guidelines. If there was an issue with dimerization, the oligonucleotides were adjusted manually. Primer BLAST was performed for verifying the specificity of the oligonucleotides against the non-redundant database and the collected beta-lactamase resistance genes. To define two multiplex PCRs, possible interactions between all oligonucleotides were analyzed with the multiple primer analyzer (Thermos Fisher Scientific). As a positive control detection of 16S rDNA was included (Nadkarni et al. 2002). The first multiplex PCR detects NDM, KPC, VIM and IMP genes and 16S rDNA, the second multiplex PCR detects OXA-23-like, OXA-40/-24-like, OXA-58-like and OXA-48-like genes.

### Bacterial isolates

To test and validate the assay, two collections from the German National Reference Laboratory (NRL) were used: one collection with all isolates from a half-month period (n=152; Jan, 21^{st} to Feb, 6^{th} 2013). The second set was a random collection with less common carbapenemase variants. The isolates were well characterized by phenotypic and genotypic testing at the NRL (Hofko et al. 2014).

In addition, bacterial isolates and the corresponding rectal swabs with a known carbapenemase were collected from the University Hospital Heidelberg during a procedure of routine patient screening at hospital admission. The isolates were regrown on BD^{™} Columbia Agar with 5 % Sheep Blood (Becton Dickinson GmbH, Heidelberg, Germany) at 37 °C.

### DNA extraction

The chelex based DNA extraction was performed as described by Martin-Platero et al. 2010. To purify the DNA, ethanol precipitation was done. Therefore, 0.3 M ammonium acetate and 80 % (v/v) of ethanol absolute was added. Samples were inverted and incubated at -80 °C for one hour. After incubation, the samples were centrifuged for 20 min at 13.300 rpm and 4 °C. The supernatant was discarded and two washing steps followed by adding 300 µl of 70 % ethanol, mixing and centrifuging for 20 min at 13.300 rpm and 4 °C. After drying the pellet, it was resuspended with 56 °C nucleic acid-free water. DNA quality was determined with the NanoDrop 1000 Spectrophotometer (Thermo Fisher Scientific); quantification was done with the Quant-iT^{™} PicoGreen^{™} dsDNA Assay Kit (Thermo Fisher Scientific).

DNA, extracted with the chelex based method, was only used to analyze the PCR performance, since a known amount of DNA is needed for determining the limit of detection. The whole validation of the assay was done with the automated extraction of the BD MAX^{™} System, by using the BD MAX^{™} ExK^{™} DNA-2 Kit.

### Multiplex PCR in PCR-only mode

Quantitative real-time PCRs were performed on the BD MAX^{™} System. For the detection of all eight carbapenemases two master mixes (MM1 and MM2) were prepared. For each reaction 12.5 µl of MM1 (2.5 µl primaQUANT 5x qPCR-Probe-MasterMix (Steinbrenner GmbH, Wiesenbach, Germany), IMP-F/R, 16S-F/R: 0.3 µM; KPC-F/R, NDM-F/R, VIM-F/R: 0.4 µM; IMP-P: 0.2 µM, 16S-P: 0.25 µM, KPC-P, NDM-P, VIM-P: 0.15 µM, 4 µl DNA, PCR-grade water) and MM2 (2.5 µl primaQUANT 5x qPCR-Probe-MasterMix, Primer: 0.4 µM, OXA-23-P, OXA-40/24-P: 0.25 µM; OXA-58-P, OXA-48-P: 0.15 µM, 4 µl DNA, PCR-grade water) were prepared. 10 µl of MM1 and MM2 were transferred to the BD MAX^{™} PCR cartridge. PCR was run in the PCR-only mode (98°C, 3 min; 98 °C, 5 s; 57 °C, 47.8 s for 3 cycles; 98 °C, 5 s; 61 °C, 43 s for 37 cycles). DNA samples were diluted to 10⁵ copies/µl and serial dilutions to one copy/µl were prepared. Copy numbers were calculated based on the measured concentration and the average genome size of each bacterial species.

### Combined extraction and PCR on the BD MAX^{™} System

The DNA was extracted with the BD MAX^{™} ExK^{™} DNA-2 Kit (4 snap configuration) from a bacterial colony of an overnight culture. MM1 and MM2 were prepared two-fold concentrated. The magnetic beads for the extraction were placed at position 1 of the extraction stripes. Position 2 and 4 were for MM1 and MM2 and at position 3 50 µl of PCR-grade water for neutralizing the elution buffer were added. One colony of an overnight culture was transferred into the sample buffer tube and vortexed before the extraction was started. The BD MAX^{™} ExK^{™} DNA-2 protocol (type 3: liquid MM with primers and probes) was used for extraction. PCR was performed with the protocol of the PCR-only mode.

### Carbapenemase detection directly from rectal swabs

Rectal swabs (ESwab^{™}, Copan) in which multidrug-resistant Gram-negative bacteria had been detected by selective culture screening were collected from the University Hospital Heidelberg and stored at - 20 °C. Swabs were selected if there was bacterial growth on a chromID^{®} ESBL plate (bioMérieux Deutschland GmbH) and if the bacteria showed resistance to meropenem and imipenem, which was tested by VITEK 2 system (bioMérieux Deutschland GmbH, Nürtingen, Germany). The routine isolates were further differentiated by multiplex SYBR green real-time PCR (Hofko et al. 2014), resulting in a known carbapenemase type. 100 µl of the rectal swab medium were transferred to the sample buffer tube of the ExK DNA-2 Kit and PCR was performed as described.

### Example 1: Primer Design

In total, 9 sets of primers and probes were designed to detect eight common carbapenemases and most of their gene variants (Table 3). Since the last update of the used sequence collection of beta-lactamases from the center for genomic epidemiology was in November 2016, it was complemented with sequences from NCBI to ensure all available variants are included. The aim was to cover at least 95 % of the carbapenemases detected during the last years in Germany by the NRL. All currently known *blaKPC* and *blaNDM* variants are detectable. The primers and probe for *blaVIM* cover all variants except *blaVIM-*7, due to high sequence variations (Fig. 4). IMP and OXA type carbapenemases show a high degree of sequence diversity that is why not all variants are detectable (Fig. 1). Hence two sets were designed to detect frequent IMP variants in Germany (Koch-Institut 2016; Pfennigwerth 2017; Koch-Institut 2015; Pfennigwerth 2018). Both sets are binding in the same gene region but the oligonucleotides differ in length, to compensate mismatches for some IMPs. Most IMPs are amplified best with either both sets or a combination of the two sets (Fig. 1, circled, *: experimentally tested variants).

**Table 3: Sequences of designed primers and probes (5'-3') for the carbapenemase detection, amplicon size and melting temperature.**

| Name of primer/probe | sequence 5'-3' | SEQ ID NO | Amplicon size | Tm [°C] |
|---|---|---|---|---|
| NDM-F | GCCACACCAGTGACAATATC | 15 | 124 bp | 62.3 |
| NDM-R | GTGCTCAGTGTCGGCAT | 16 | | 62.5 |
| NDM-P | ACTTGGCCTTGCTGTCCTTGATCA | 17 | | 68.8 |
| KPC-F | CAGCTCATTCAAGGGCTT | 38 | 148 bp | 60.5 |
| KPC-R | CGTCATGCCTGTTGTCAG | 39 | | 61.0 |
| KPC-P | CACACCCATCCGTTACGGCAA | 40 | | 67.4 |
| VIM-F | TCCAATGGTCTCATTGTCC | 59 | 145 bp | 60.0 |
| VIM-R | CATGAAAGTGCGTGGAGA | 60 | | 60.3 |
| VIM-P | ATGAGTTGCTTTTGATTGATACAGCKTGG | 61 | | 67.2-68.4 |
| IMP-F1 | GGAATAGAGTGGCTTAATTCTC | 73 | 190 bp | 59.0 |
| IMP-R1 | GCCAAACCACTACGTTATCTK | 74 | | 61.6-61.9 |
| IMP-P1 | ATGCRTCTGAATTAACAAATGARCTTCT | 75 | | 64.0-66.7 |
| IMP-F2 | GGTGGAATAGAGTGGCTTAATTCTC | 76 | 193 bp | 63.4 |
| IMP-R2 | GCCAAACCACTACGTTATCTKGAG | 77 | | 64.2-65.5 |
| IMP-P2 | CCCACGTATGCRTCTGAATTAACAAATGAR CTTCT | 78 | | 69.5-71.4 |
| OXA-23-like-F | TAAATGGAAGGGCGAGAA | 104 | 180 bp | 59.0 |
| OXA-23-like-R | ACCTGCTGTCCAATTTCAG | 105 | | 60.8 |
| OXA-23-like-P | CCATGAAGCTTTCTGCAGTCCCAGTC | 106 | | 69.0 |
| OXA-48-like-F | AGGGCGTAGTTGTGCTC | 121 | 149 bp | 61.9 |
| OXA-48-like-R | GTGTTCATCCTTAACCACGC | 122 | | 61.7 |
| OXA-48-like-P | TCTTAAACGGGCGAACCAAGCAT | 123 | | 67.5 |
| OXA-58-like-F | ATTGGCACGTCGTATTGG | 133 | 164 bp | 60.7 |
| OXA-58-like-R | CCCCTCTGCGCTCTACATA | 134 | | 63.2 |
| OXA-58-like-P | AGTGAATTGCAACGTATTGGTTATGGCA | 135 | | 68.1 |
| OXA-40/24-like-F | TGACTTTAGGTGAGGCAATG | 143 | 212 bp | 60.6 |
| OXA-40/24-like-R | GTTATGTGCAAGGTCATCGG | 144 | | 61.5 |
| OXA-40/24-like-P | TGCAAGACGGACTGGCCTAGAGCTAAT | 145 | | 70.6 |
| 16S-F | TCCTACGGGAGGCAGCAGT | 147 | 466 bp | 59.4 |
| 16S-R | GGACTACCAGGGTATCTAATCCTGTT | 148 | | 58.1 |
| 16S-P | CGTATTACCGCGGCTGCTGGCAC | 149 | | 69.9 |

| | | | | |
|---|---|---|---|---|
| NDM-P comprises a 5' FAM moiety and a 3' BHQ1 moiety. KPC-P comprises a 5' JOE moiety and a 3' BHQ1 moiety. VIM-P comprises a 5' TexasRed moiety and a 3' BHQ2 moiety. IMP-P1 and IMP-P2 comprise a 5' Cy5 moiety and a 3' BHQ2 moiety. OXA-23-like-P comprises a 5' FAM moiety and a 3' BHQ1 moiety. OXA-72-like-P comprises a 5' JOE moiety and a 3' BHQ1 moiety. OXA-58-like-P comprises a 5' TexasRed moiety and a 3' BHQ2 moiety. OXA-48-like-P comprises a 5' Cy5 moiety and a 3' BHQ2 moiety. 16S-P comprises a 5' Cy5.5 moiety and a 3' BBQ moiety (Nadkarni et al. 2002). | | | | |

OXA beta-lactamases have a different spectrum of activity. OXA-23-like, OXA-58-like, OXA-40/24-like, OXA-48-like and OXA-51-like are able to hydrolyze carbapenems. The other OXA-groups are extended-spectrum, broad-spectrum or narrow-spectrum beta-lactamases (Antunes and Fisher 2014). Primers and hydrolysis probes were designed for OXA-23-like, OXA-58-like, OXA-40/24-like and OXA-48-like (Fig. 1B, colored tip labels). OXA-51-like carbapenemases that commonly occur chromosomally in *Acinetobacter* species were not included in the assay. OXA-51-like genes have a high degree of sequence diversity, thus more than one set of primers and probes would have been needed to cover all or most variants of this carbapenemase (Fig. 1B; (Evans et al. 2008)). Furthermore not all *Acinetobacter* harboring only an OXA-51-like carbapenemase are resistant to carbapenems. (Turton et al. 2006).

Based on the data of the NRL in Germany in the years 2014 - 2017, 98.2 % of the carbapenemases in *Enterobacteriaceae,* 94.0 % in *P. aeruginosa* and 96.1 % in *A. baumannii* are theoretically detectable with this assay, which leads to an overall theoretical sensitivity of 97.1 %.

### Example 2: Assay Performance

The assay performance was determined with isolated DNA in the PCR-only mode on the BD MAX^{™} System. All eight carbapenemases and tested variants are detectable with this assay. The oligonucleotides were specific, as there was neither amplification of ESBLs (CTX-M, GES, SHV, TEM) and AmpC beta-lactamases (DHA, CMY, ACT) nor of beta-lactamase negative bacteria. Additionally, different variants of a carbapenemase were tested with the PCR and were all identified correctly (Table 4). The PCR showed 100 % sensitivity and 100 % specificity with the tested isolates.

**Table 4: Tested carbapenemase variants with the PCR-only mode**

| Carbapenemase | gene variants |
|---|---|
| NDM | NDM-1, NDM-1/-6, NDM-2 |
| KPC | KPC-2, KPC-3 |
| VIM | VIM-1, VIM-2, VIM-4, VIM-5, VIM-26, VIM-28 |
| IMP | IMP-1, IMP-2/-19, IMP-4/-28, IMP-7, IMP-8/-24, IMP-13, IMP-14, IMP-15, IMP-16 |
| OXA-23-like | OXA-23 |
| OXA-40/24-like | OXA-72 |
| OXA-58-like | OXA-58 |
| OXA-48-like | OXA-48, OXA-162, OXA-181, OXA-204 |

To define the limit of detection (LOD), one representative target of each carbapenemase was chosen (NDM-1/6, KPC-3, VIM-1, IMP-8/24, OXA-23, OXA-72, OXA-58, OXA-48). PCR was done with serial dilutions in four replicates. For NDM, VIM, OXA-23, OXA-72, OXA-58 and OXA-48 the detection of 3.2 copies/PCR was possible. The LOD for KPC and IMP was 32 copies.

Based on the standard, PCR efficiency was calculated. The desired range for PCR efficiencies is between 90 % and 110 %, which is the case for NDM, OXA-58, OXA-72 and OXA-48. KPC and OXA-23 are with 88 % and 89 % almost in this range. Only VIM-1 and IMP-8/24 showed poorer efficiencies with 80 % and 77 % (Fig. 2).

### Example 3: Validation of the automated assay

The assay was validated as a fully automated PCR with consecutive isolates collected at the NRL from January, 21^{st} to February, 6^{th} 2013. A total of 152 isolates were tested with the BD MAX^{™} ExK^{™} DNA-2 extraction kit. The collection contains 59 carbapenemase positive strains (NDM: n=5, KPC: n=8, VIM: n=23, OXA-23: n=12, OXA-48: n=10, NDM and OXA-48: n=1), 12 isolates with ESBLs and AmpC resistance genes (GES: n=5, CMY: n=5, SHV: n=1, DHA: n=1) and 81 beta-lactamase negative isolates. Testing was done by inoculation of sample material from grown isolates into the BD MAX^{™} extraction tube.

All 59 carbapenemases were identified correctly. The PCR was considered as successful if either a signal for the positive control or for one of the carbapenemases was detectable. The isolates harboring ESBLs and AmpC beta-lactamases were detected as negatives. For six isolates, the PCR was repeated once, since there was neither a signal for one of the carbapenemases nor for 16S positive control. 80 of the 81 beta-lactamase negative isolates were negative. For one beta-lactamase negative isolate the detection of the positive control failed, even after repetition (Table 5). Only few isolates showed some unspecific OXA-23-like amplification in mastermix 2 with a Ct value higher 31.0 and a low fluorescence signal, which was considered as background noise, as testing from bacterial colonies leads to clear signals (Ct < 25) for carbapenemase positive isolates.

**Table 5: Validation of the assay with a consecutive collection of 152 isolates from the NRL, collected from January, 21st to February, 6th 2013. GES, CMY, DHA and SHV were considered as "negative".**

| Beta-lactamase NRL result | Bacterial species ^{a} | Number of isolates | BD MAX^{™} result | concordant | discordant |
|---|---|---|---|---|---|
| VIM-1 | klox, encl, klpn, esco | 13 | VIM | 13 | 0 |
| VIM-2 | psae, | 10 | VIM | 10 | 0 |
| KPC-2 | klpn, | 4 | KPC | 4 | 0 |
| KPC-3 | klpn | 4 | KPC | 4 | 0 |
| NDM-1/6 | esco, klpn, acba | 5 | NDM | 5 | 0 |
| NDM-1/6, OXA-23 | acba | 1 | NDM, OXA-23-like | 1 | 0 |
| OXA-23 | acba, | 12 | OXA-23-like | 12 | 0 |
| OXA-23 | acba, | 12 | OXA-23-like | 12 | 0 |
| OXA-48 | klpn, esco, | 9 | OXA-48-like | 9 | 0 |
| GES-1 | psae, | 5 | negative | 5 | 0 |
| CMY-2 | esco, prmi | 3 | negative | 3 | 0 |
| CMY-2/22 | prmi | 2 | negative | 2 | 0 |
| SHV-12 | encl, | 1 | negative | 1 | 0 |
| DHA | esco | 1 | negative | 1 | 0 |
| Negative | psae, encl, klpn, enae, esco, acba, prmi, momo, acpi, acju, xama, cifr, pssp | 81 | negative | 80 | 1 |

| | | | | | |
|---|---|---|---|---|---|
| ^{a} acba: *Acinetobacter baumannii*, acpi: *Acinetobacter pittii*, acju: *Acinetobacter junii*, cifr: *Citrobacter freundii*, esco: *Escherichia coli*, enae: *Enterobacter aerogenes*, encl: *Enterobacter cloacae*, klpn: *Klebsiella pneumoniae*, klox: *Klebsiella oxytoca*, momo: *Morganella morganii*, psae: *Pseudomonas aeruginosa*, pssp: *Pseudomonas sp*., prmi: *Proteus mirabilis*, xama: *Xanthomonas maltophila* | | | | | |

For further validation, isolates with less common carbapenemase variants, which were not present in the consecutive collection, were tested. Different OXA (OXA-58, OXA-72, OXA-204) and IMP (IMP-1, IMP-13, IMP-16, IMP-7, IMP-14, IMP-15, IMP-4/28, IMP-2/19) variants were identified correctly as well as isolates that harbor two carbapenemases (KPC-2+VIM-26, NDM-1/6+OXA-48, OXA-23+OXA-58, Table 6).

**Table 6: Further validation of the assay with less common carbapenemase variants.**

| Beta-lactamase NRL/UKHD result | Bacterial species^{a} | Number of isolates | BD MAX^{™} result | concordant | discordant |
|---|---|---|---|---|---|
| OXA-58 | acba | 1 | OXA-58-like | 1 | 0 |
| OXA-72 | acba | 1 | OXA-40/24-like | 1 | 0 |
| OXA-204 | klpn | 1 | OXA-48-like | 1 | 0 |
| IMP-13 | acba | 1 | IMP | 1 | 0 |
| IMP-1 | psae | 1 | IMP | 1 | 0 |
| IMP-16 | psae | 1 | IMP | 1 | 0 |
| IMP-7 | psae | 1 | IMP | 1 | 0 |
| IMP-14 | encl | 1 | IMP | 1 | 0 |
| IMP-15 | psae | 1 | IMP | 1 | 0 |
| IMP-4/-28 | encl | 1 | IMP | 1 | 0 |
| IMP-2/19 | psae | 1 | IMP | 1 | 0 |
| KPC-2, VIM-26 | klpn | 1 | KPC, VIM | 1 | 0 |
| NDM-1/-6, OXA-48 | klpn | 1 | NDM, OXA-48-like | 1 | 0 |
| OXA-23, OXA-58 | acba | 1 | OXA-23-like, OXA-58-like | 1 | 0 |

| | | | | | |
|---|---|---|---|---|---|
| *a acba: Acinetobacter baumannii, encl: Enterobacter cloacae, klpn: Klebsiella pneumoniae, psae: Pseudomonas aeruginosa* | | | | | |

### Example 4: Carbapenemase detection directly from rectal swabs

Next to the use of the automated PCR for bacterial isolates, direct detection from rectal swabs, e.g. during screening procedures, might be envisioned. Thus, rectal swabs were collected from routine diagnostics at the University Hospital Heidelberg if there was growth on chromID^{®} ESBL plates and if the isolate showed resistance to carbapenems. Due to differentiation with the multiplex SYBR green real-time PCR (Hofko et al. 2014) the carbapenemase type was known.

A total of 79 genes were detected either in the differentiated isolate or in the direct PCR samples. 60 of 79 genes were concordant. 11 of 79 genes could be detected in the direct PCR-sample but were not identified in routine diagnostics. Thus, in individual cases direct testing was more sensitive than culture-based methods. There was no growth on chromID^{®} ESBL plates but a clear fluorescence signal for OXA-48, indicating that the bacteria perhaps were not viable/ cultivable anymore due to antibiotic treatment or unculturability of the carrier. 8 of 79 genes led to discordant results, since carbapenemases were detected in the differentiated isolate but not in the direct PCR sample (Fig. 3, Table 7).

**Table 7: Comparison of direct PCR-samples to differentiated isolates**

| Number of rectal swab | Cultured isolate | Result of direct PCR in rectal swab | concordant | detection of new carbapenemase in direct PCR | discordant |
|---|---|---|---|---|---|
| 1 | OXA-23-like | OXA-23-like | 1 | 0 | 0 |
| 2 | OXA-48-like | OXA-48-like | 1 | 0 | 0 |
| 3 | OXA-48-like | OXA-48-like | 1 | 0 | 0 |
| 4 | OXA-23-like | OXA-23-like | 1 | 0 | 0 |
| 5 | KPC | KPC | 1 | 0 | 0 |
| 6 | OXA-48-like | OXA-48-like | 1 | 0 | 0 |
| 7 | OXA-48-like | OXA-48-like | 1 | 0 | 0 |
| 8 | OXA-48-like | OXA-48-like | 1 | 0 | 0 |
| 9 | NDM, OXA-48-like | NDM, OXA-48-like, OXA-23-like | 2 | 1 | 0 |
| 10 | NDM | NDM, OXA-23-like, OXA-48-like | 1 | 2 | 0 |
| 11 | OXA-48-like | OXA-48-like | 1 | 0 | 0 |
| 12 | OXA-48-like | OXA-48-like | 1 | 0 | 0 |
| 13 | OXA-48-like | OXA-48-like | 1 | 0 | 0 |
| 14 | OXA-48-like | OXA-48-like | 1 | 0 | 0 |
| 15 | OXA-48-like | OXA-48-like | 1 | 0 | 0 |
| 16 | VIM-1 | VIM | 1 | 0 | 0 |
| 17 | VIM-1 | VIM | 1 | 0 | 0 |
| 18 | NDM, OXA-48-like | NDM, OXA-48-like | 2 | 0 | 0 |
| 19 | KPC | KPC | 1 | 0 | 0 |
| 20 | OXA-48-like | OXA-48-like | 1 | 0 | 0 |
| 21 | OXA-48-like | OXA-48-like | 1 | 0 | 0 |
| 22 | OXA-48-like | OXA-48-like | 1 | 0 | 0 |
| 23 | OXA-48-like | OXA-48-like | 1 | 0 | 0 |
| 24 | OXA-48-like | OXA-48-like | 1 | 0 | 0 |
| 25 | KPC | KPC, OXA-23-like, NDM | 1 | 2 | 0 |
| 26 | OXA-48-like | OXA-48-like | 1 | 0 | 0 |
| 27 | OXA-48-like | OXA-48-like | 1 | 0 | 0 |
| 28 | OXA-48-like | OXA-48-like | 1 | 0 | 0 |
| 29 | OXA-48-like | OXA-48-like | 1 | 0 | 0 |
| 30 | VIM-2 | VIM | 1 | 0 | 0 |
| 31 | KPC | KPC | 1 | 0 | 0 |
| 32 | NDM | NDM | 1 | 0 | 0 |
| 33 | OXA-48-like | OXA-48-like | 1 | 0 | 0 |
| 34 | NDM | NDM | 1 | 0 | 0 |
| 35 | NDM | NDM | 1 | 0 | 0 |
| 36 | NDM | NDM | 1 | 0 | 0 |
| 37 | OXA-48-like | OXA-48 | 1 | 0 | 0 |
| 38 | NDM | NDM | 1 | 0 | 0 |
| 39 | NDM | NDM | 1 | 0 | 0 |
| 40 | OXA-48-like | OXA-48-like | 1 | 0 | 0 |
| 41 | KPC | KPC | 1 | 0 | 0 |
| 42 | OXA-48-like | OXA-48-like | 1 | 0 | 0 |
| 43 | VIM-1 | VIM | 1 | 0 | 0 |
| 44 | VIM-2 | VIM | 1 | 0 | 0 |
| 45 | VIM-2 | VIM | 1 | 0 | 0 |
| 46 | OXA-48-like | OXA-48-like | 1 | 0 | 0 |
| 47 | OXA-23-like | OXA-23-like | 1 | 0 | 0 |
| 48 | OXA-48-like | OXA-48-like | 1 | 0 | 0 |
| 49 | KPC | KPC | 1 | 0 | 0 |
| 50 | NDM | NDM | 1 | 0 | 0 |
| 51 | OXA-48-like | OXA-48-like | 1 | 0 | 0 |
| 52 | OXA-48-like | OXA-48-like | 1 | 0 | 0 |
| 53 | OXA-48-like | OXA-48-like | 1 | 0 | 0 |
| 54 | OXA-48-like | OXA-48-like | 1 | 0 | 0 |
| 55 | NDM | NDM, OXA-48-like | 1 | 1 | 0 |
| 56 | NDM | NDM, OXA-48-like | 1 | 1 | 0 |
| 57 | KPC | KPC, VIM, OXA-23-like | 1 | 2 | 0 |
| 58 | OXA-23-like | OXA-48-like | 0 | 1 | 1 |
| 59 | OXA-48-like | OXA-48-like, OXA-23-like | 1 | 1 | 0 |
| 60 | VIM-1 | negative | 0 | 0 | 1 |
| 61 | VIM-1 | negative | 0 | 0 | 1 |
| 62 | KPC | negative | 0 | 0 | 1 |
| 63 | KPC | negative | 0 | 0 | 1 |
| 64 | IMP-1 | negative | 0 | 0 | 1 |
| 65 | VIM-1 | negative | 0 | 0 | 1 |
| 66 | NDM | inhibition | 0 | 0 | 1 |

### References

Antonelli, A., F. Arena, T. Giani, O. L. Colavecchio, S. V. Valeva, S. Paule, P. Boleij, and G. M. Rossolini. 2016. 'Performance of the BD MAX instrument with Check-Direct CPE real-time PCR for the detection of carbapenemase genes from rectal swabs, in a setting with endemic dissemination of carbapenemase-producing Enterobacteriaceae', Diagn Microbiol Infect Dis, 86: 30-4.
Antunes, N. T., and J. F. Fisher. 2014. 'Acquired Class D beta-Lactamases', Antibiotics (Basel), 3: 398-434.
Chavda, K. D., L. Chen, M. R. Jacobs, A. D. Rojtman, R. A. Bonomo, and B. N. Kreiswirth. 2015. 'Complete sequence of a bla(KPC)-harboring cointegrate plasmid isolated from Escherichia coli', Antimicrob Agents Chemother, 59: 2956-9.
Cuzon, G., T. Naas, P. Bogaerts, Y. Glupczynski, and P. Nordmann. 2012. 'Evaluation of a DNA microarray for the rapid detection of extended-spectrum beta-lactamases (TEM, SHV and CTX-M), plasmid-mediated cephalosporinases (CMY-2-like, DHA, FOX, ACC-1, ACT/MIR and CMY-1-like/MOX) and carbapenemases (KPC, OXA-48, VIM, IMP an accession number d NDM)', J Antimicrob Chemother, 67: 1865-9.
Dallenne, C., A. Da Costa, D. Decre, C. Favier, and G. Arlet. 2010. 'Development of a set of multiplex PCR assays for the detection of genes encoding important beta-lactamases in Enterobacteriaceae', J Antimicrob Chemother, 65: 490-5.
Doumith, M., M. J. Ellington, D. M. Livermore, and N. Woodford. 2009. 'Molecular mechanisms disrupting porin expression in ertapenem-resistant Klebsiella and Enterobacter spp. clinical isolates from the UK', J Antimicrob Chemother, 63: 659-67.
El Amin, N., C. G. Giske, S. Jalal, B. Keijser, G. Kronvall, and B. Wretlind. 2005. 'Carbapenem resistance mechanisms in Pseudomonas aeruginosa: alterations of porin OprD and efflux proteins do not fully explain resistance patterns observed in clinical isolates', APMIS, 113: 187-96.
Evans, B. A., A. Hamouda, K. J. Towner, and S. G. Amyes. 2008. 'OXA-51-like beta-lactamases and their association with particular epidemic lineages of Acinetobacter baumannii', Clin Microbiol Infect, 14: 268-75.
Hofko, M., A. Mischnik, M. Kaase, S. Zimmermann, and A. H. Dalpke. 2014. 'Detection of carbapenemases by real-time PCR and melt curve analysis on the BD Max system', J Clin Microbiol, 52: 1701-4.
Kaase, M. 2012. '[Carbapenemases in gram-negative bacteria. Current data and trends of resistance resulting from the work of national reference centres]', Bundesgesundheitsblatt Gesundheitsforschung Gesundheitsschutz, 55: 1401-4.
Koch-Institut, Robert. 2015. "Bericht des Nationalen Referenzzentrums (NRZ) für gramnegative Krankenhauserreger." In.: Robert Koch-Institut, Epidemiologie und Gesundheitsberichterstattung.
Koch-Institut, Robert. 2016. "Bericht des Nationalen Referenzzentrums (NRZ) für gramnegative Krankenhauserreger." In.: Robert Koch-Institut, Epidemiologie und Gesundheitsberichterstattung.
Lund, M., M. B. Petersen, A. L. Jorgensen, D. Paulmann, and M. Wang. 2018. 'Rapid real-time PCR for the detection of IMP, NDM, VIM, KPC and OXA-48 carbapenemase genes in isolates and spiked stool samples', Diagn Microbiol Infect Dis, 92: 8-12.
Mentasti, M., K. Prime, K. Sands, S. Khan, and M. Wootton. 2019. 'Rapid detection of IMP, NDM, VIM, KPC and OXA-48-like carbapenemases from Enterobacteriales and Gram-negative non-fermenter bacteria by real-time PCR and melt-curve analysis', Eur J Clin Microbiol Infect Dis.
Naas, T., G. Cuzon, P. Bogaerts, Y. Glupczynski, and P. Nordmann. 2011. 'Evaluation of a DNA microarray (Check-MDR CT102) for rapid detection of TEM, SHV, and CTX-M extended-spectrum beta-lactamases and of KPC, OXA-48, VIM, IMP, and NDM-1 carbapenemases', J Clin Microbiol, 49: 1608-13.
Naas, T., and P. Nordmann. 1994. 'Analysis of a carbapenem-hydrolyzing class A beta-lactamase from Enterobacter cloacae and of its LysR-type regulatory protein', Proc Natl Acad Sci U S A, 91: 7693-7.
Nadkarni, M. A., F. E. Martin, N. A. Jacques, and N. Hunter. 2002. 'Determination of bacterial load by real-time PCR using a broad-range (universal) probe and primers set', Microbiology, 148: 257-66.
Pfennigwerth, Niels. 2017. "Bericht des Nationalen Referenzzentrums (NRZ) für gramnegative Krankenhauserreger." In.: Robert Koch-Institut, Epidemiologie und Gesundheitsberichterstattung.
Koch-Institut Robert. 2018. "Bericht des Nationalen Referenzzentrums (NRZ) für gramnegative Krankenhauserreger." In.: Robert Koch-Institut, Epidemiologie und Gesundheitsberichterstattung.
Queenan, A. M., and K. Bush. 2007. 'Carbapenemases: the versatile beta-lactamases', Clin Microbiol Rev, 20: 440-58, table of contents.
Saliba, R., C. Neulier, D. Seytre, A. Fiacre, F. Faibis, P. Leduc, M. Amara, F. Jaureguy, E. Carbonnelle, J. R. Zahar, and L. Marty. 2019. 'Can real-time polymerase chain reaction allow a faster recovery of hospital activity in cases of an incidental discovery of carbapenemase-producing Enterobacteriaceae and vancomycin-resistant Enterococci carriers?', J Hosp Infect.
Stoesser, N., A. E. Sheppard, G. Peirano, L. W. Anson, L. Pankhurst, R. Sebra, H. T. T. Phan, A. Kasarskis, A. J. Mathers, T. E. A. Peto, P. Bradford, M. R. Motyl, A. S. Walker, D. W. Crook, and J. D. Pitout. 2017. 'Genomic epidemiology of global Klebsiella pneumoniae carbapenemase (KPC)-producing Escherichia coli', Sci Rep, 7: 5917.
Teo, J. W., M. V. La, and R. T. Lin. 2016. 'Development and evaluation of a multiplex real-time PCR for the detection of IMP, VIM, and OXA-23 carbapenemase gene families on the BD MAX open system', Diagn Microbiol Infect Dis, 86: 358-61.
Teo, J. W., S. Octavia, J. W. Cheng, and R. T. Lin. 2018. 'Evaluation of an in-house developed multiplex real-time PCR for the detection of IMP, OXA-23, GES carbapenemases and the transmissible colistin-resistant mcr gene on the BD MAX open system', Diagn Microbiol Infect Dis, 90: 67-69.
Turton, J. F., M. E. Ward, N. Woodford, M. E. Kaufmann, R. Pike, D. M. Livermore, and T. L. Pitt. 2006. 'The role of ISAba1 in expression of OXA carbapenemase genes in Acinetobacter baumannii', FEMS Microbiol Lett, 258: 72-7.

## Claims

1. A method for determining in a sample the presence or absence of carbapenemase producing bacteria, the method comprising determining in said sample the presence or absence of one or more carbapenemase genes selected from the group consisting of
a) an NDM carbapenemase gene, the presence of which is determined using a forward primer F1, a reverse primer R1 and optionally a probe P1, wherein F1, R1 and P1 are each capable of hybridization to SEQ ID NO:1 or its complement;
b) a KPC carbapenemase gene, the presence of which is determined using a forward primer F2, a reverse primer R2 and optionally a probe P2, wherein F2, R2 and P2 are each capable of hybridization to SEQ ID NO:27 or its complement;
c) a VIM carbapenemase gene, the presence of which is determined using a forward primer F3, a reverse primer R3 and optionally a probe P3, wherein F3, R3 and P3 are each capable of hybridization to SEQ ID NO:47 or its complement;
d) an IMP carbapenemase gene, the presence of which is determined using a forward primer F4, a reverse primer R4 and optionally a probe P4, wherein F4, R4 and P4 are each capable of hybridization to SEQ ID NO:69 or its complement, SEQ ID NO:79 or its complement, or SEQ ID NO:86 or its complement;
e) an OXA-23-like carbapenemase gene, the presence of which is determined using a forward primer F5, a reverse primer R5 and optionally a probe P5, wherein F5, R5 and P5 are each capable of hybridization to SEQ ID NO:93 or its complement;
f) an OXA-48-like carbapenemase gene, the presence of which is determined using a forward primer F6, a reverse primer R6 and optionally a probe P6, wherein F6, R6 and P6 are each capable of hybridization to SEQ ID NO:113 or its complement;
g) an OXA-58-like carbapenemase gene, the presence of which is determined using a forward primer F7, a reverse primer R7 and optionally a probe P7, wherein F7, R7 and P7 are each capable of hybridization to SEQ ID NO:127 or its complement; and
h) an OXA-40/24-like carbapenemase gene, the presence of which is determined using a forward primer F8, a reverse primer R8 and optionally a probe P8, wherein F8, R8 and P8 are each capable of hybridization to SEQ ID NO:138 or its complement.

2. The method according to claim 1, comprising the steps of
a. amplification of a region of bacterial DNA comprised in the sample using at least one primer pair consisting of a forward and a corresponding reverse primer of claim 1; and
b. detection of the presence or absence of an amplicon,
wherein the presence of an amplicon is indicative for the presence of carbapenemase producing bacteria in the sample and the absence of an amplicon is indicative for the absence of carbapenemase producing bacteria in the sample.

3. The method according to claim 1 or 2, wherein the presence or absence of at least one or at least two carbapenemase genes selected from the group consisting of an IMP carbapenemase gene, an OXA-23-like carbapenemase gene, an OXA-40/24-like carbapenemase gene and an OXA-58-like carbapenemase gene is determined, preferably wherein the presence or absence of at least one carbapenemase gene selected from the group consisting of an OXA-40/24-like carbapenemase gene and an OXA-58-like carbapenemase gene is determined.

4. The method according to any one of claims 1 to 3, wherein
a) F1, R1 and P1 are each capable of hybridization to SEQ ID NO:2 or its complement;
b) F2, R2 and P2 are each capable of hybridization to SEQ ID NO:28 or its complement;
c) F3, R3 and P3 are each capable of hybridization to SEQ ID NO:48 or its complement;
d) F4, R4 and P4 are each capable of hybridization to SEQ ID NO:69 or its complement;
e) F5, R5 and P5 are each capable of hybridization to SEQ ID NO:94 or its complement;
f) F6, R6 and P6 are each capable of hybridization to SEQ ID NO:114 or its complement;
g) F7, R7 and P7 are each capable of hybridization to SEQ ID NO:127 or its complement; and/or
h) F8, R8 and P8 are each capable of hybridization to SEQ ID NO:138 or its complement.

5. The method according to any one of claims 1 to 4, wherein
a) the sequence of F1 comprises or consists of 16 to 30 consecutive nucleotides of SEQ ID NO:3, the sequence of R1 comprises or consists of 16 to 30 consecutive nucleotides of SEQ ID NO:4 and the sequence of P1 comprises or consists of 20 to 30 consecutive nucleotides of SEQ ID NO:5; or the sequence of F1 comprises or consists of 16 to 30 consecutive nucleotides of SEQ ID NO:6, the sequence of R1 comprises or consists of 16 to 30 consecutive nucleotides of SEQ ID NO:7 and the sequence of P1 comprises or consists of 20 to 30 consecutive nucleotides of SEQ ID NO:8; or the sequence of F1 comprises or consists of 16 to 30 consecutive nucleotides of SEQ ID NO:9, the sequence of R1 comprises or consists of 16 to 30 consecutive nucleotides of SEQ ID NO:10 and the sequence of P1 comprises or consists of 20 to 30 consecutive nucleotides of SEQ ID NO:11; or the sequence of F1 comprises or consists of 16 to 30 consecutive nucleotides of SEQ ID NO:12, the sequence of R1 comprises or consists of 16 to 30 consecutive nucleotides of SEQ ID NO:13 and the sequence of P1 comprises or consists of 20 to 30 consecutive nucleotides of SEQ ID NO:14;
b) the sequence of F2 comprises or consists of 16 to 30 consecutive nucleotides of SEQ ID NO:29, the sequence of R2 comprises or consists of 16 to 30 consecutive nucleotides of SEQ ID NO:30 and the sequence of P2 comprises or consists of 20 to 30 consecutive nucleotides of SEQ ID NO:31; or the sequence of F2 comprises or consists of 16 to 30 consecutive nucleotides of SEQ ID NO:32, the sequence of R2 comprises or consists of 16 to 30 consecutive nucleotides of SEQ ID NO:33 and the sequence of P2 comprises or consists of 20 to 30 consecutive nucleotides of SEQ ID NO:34; or the sequence of F2 comprises or consists of 16 to 30 consecutive nucleotides of SEQ ID NO:35, the sequence of R2 comprises or consists of 16 to 30 consecutive nucleotides of SEQ ID NO:36 and the sequence of P2 comprises or consists of 20 to 30 consecutive nucleotides of SEQ ID NO:37;
c) the sequence of F3 comprises or consists of 16 to 30 consecutive nucleotides of SEQ ID NO:49, the sequence of R3 comprises or consists of 16 to 30 consecutive nucleotides of SEQ ID NO:50 and the sequence of P3 comprises or consists of 20 to 30 consecutive nucleotides of SEQ ID NO:51; or the sequence of F3 comprises or consists of 16 to 30 consecutive nucleotides of SEQ ID NO:52, the sequence of R3 comprises or consists of 16 to 30 consecutive nucleotides of SEQ ID NO:53 and the sequence of P3 comprises or consists of 20 to 30 consecutive nucleotides of SEQ ID NO:54; or the sequence of F3 comprises or consists of 16 to 30 consecutive nucleotides of SEQ ID NO:55, the sequence of R3 comprises or consists of 16 to 30 consecutive nucleotides of SEQ ID NO:56 or SEQ ID NO:57 and the sequence of P3 comprises or consists of 20 to 30 consecutive nucleotides of SEQ ID NO:58;
d) the sequence of F4 comprises or consists of 16 to 30 consecutive nucleotides of SEQ ID NO:70, the sequence of R4 comprises or consists of 16 to 30 consecutive nucleotides of SEQ ID NO:71 and the sequence of P4 comprises or consists of 20 to 30 consecutive nucleotides of SEQ ID NO:72; or the sequence of F4 comprises or consists of 16 to 30 consecutive nucleotides of SEQ ID NO:80, the sequence of R4 comprises or consists of 16 to 30 consecutive nucleotides of SEQ ID NO:81 and the sequence of P4 comprises or consists of 20 to 30 consecutive nucleotides of SEQ ID NO:82; or the sequence of F4 comprises or consists of 16 to 30 consecutive nucleotides of SEQ ID NO:87, the sequence of R4 comprises or consists of 16 to 30 consecutive nucleotides of SEQ ID NO:88 and the sequence of P4 comprises or consists of 20 to 30 consecutive nucleotides of SEQ ID NO:89;
e) the sequence of F5 comprises or consists of 16 to 30 consecutive nucleotides of SEQ ID NO:95, the sequence of R5 comprises or consists of 16 to 30 consecutive nucleotides of SEQ ID NO:96 and the sequence of P5 comprises or consists of 20 to 30 consecutive nucleotides of SEQ ID NO:97; or the sequence of F5 comprises or consists of 16 to 30 consecutive nucleotides of SEQ ID NO:98, the sequence of R5 comprises or consists of 16 to 30 consecutive nucleotides of SEQ ID NO:99 and the sequence of P5 comprises or consists of 20 to 30 consecutive nucleotides of SEQ ID NO:100; or the sequence of F5 comprises or consists of 16 to 30 consecutive nucleotides of SEQ ID NO:101, the sequence of R5 comprises or consists of 16 to 30 consecutive nucleotides of SEQ ID NO:102 and the sequence of P5 comprises or consists of 20 to 30 consecutive nucleotides of SEQ ID NO:103;
f) the sequence of F6 comprises or consists of 16 to 30 consecutive nucleotides of SEQ ID NO:115, the sequence of R6 comprises or consists of 16 to 30 consecutive nucleotides of SEQ ID NO:116 and the sequence of P6 comprises or consists of 20 to 30 consecutive nucleotides of SEQ ID NO:117; or the sequence of F6 comprises or consists of 16 to 30 consecutive nucleotides of SEQ ID NO:118, the sequence of R6 comprises or consists of 16 to 30 consecutive nucleotides of SEQ ID NO:119 and the sequence of P6 comprises or consists of 20 to 30 consecutive nucleotides of SEQ ID NO:120;
g) the sequence of F7 comprises or consists of 16 to 30 consecutive nucleotides of SEQ ID NO:128 or SEQ ID NO:131, the sequence of R7 comprises or consists of 16 to 30 consecutive nucleotides of SEQ ID NO:129 and the sequence of P7 comprises or consists of 20 to 30 consecutive nucleotides of SEQ ID NO:130 or SEQ ID NO:132; and/or
h) the sequence of F8 comprises or consists of 16 to 30 consecutive nucleotides of SEQ ID NO:139, the sequence of R8 comprises or consists of 16 to 30 consecutive nucleotides of SEQ ID NO:140 and the sequence of P8 comprises or consists of 20 to 30 consecutive nucleotides of SEQ ID NO:141 or SEQ ID NO:142.

6. The method according to any one of claims 1 to 5, wherein
a) the sequence of F1 comprises or consists of 16 to 30 consecutive nucleotides of SEQ ID NO:3, the sequence of R1 comprises or consists of 16 to 30 consecutive nucleotides of SEQ ID NO:4 and the sequence of P1 comprises or consists of 20 to 30 consecutive nucleotides of SEQ ID NO:5;
b) the sequence of F2 comprises or consists of 16 to 30 consecutive nucleotides of SEQ ID NO:29, the sequence of R2 comprises or consists of 16 to 30 consecutive nucleotides of SEQ ID NO:30 and the sequence of P2 comprises or consists of 20 to 30 consecutive nucleotides of SEQ ID NO:31;
c) the sequence of F3 comprises or consists of 16 to 30 consecutive nucleotides of SEQ ID NO:49, the sequence of R3 comprises or consists of 16 to 30 consecutive nucleotides of SEQ ID NO:50 and the sequence of P3 comprises or consists of 20 to 30 consecutive nucleotides of SEQ ID NO:51;
d) the sequence of F4 comprises or consists of 16 to 30 consecutive nucleotides of SEQ ID NO:70, the sequence of R4 comprises or consists of 16 to 30 consecutive nucleotides of SEQ ID NO:71 and the sequence of P4 comprises or consists of 20 to 30 consecutive nucleotides of SEQ ID NO:72;
e) the sequence of F5 comprises or consists of 16 to 30 consecutive nucleotides of SEQ ID NO:95, the sequence of R5 comprises or consists of 16 to 30 consecutive nucleotides of SEQ ID NO:96 and the sequence of P5 comprises or consists of 20 to 30 consecutive nucleotides of SEQ ID NO:97;
f) the sequence of F6 comprises or consists of 16 to 30 consecutive nucleotides of SEQ ID NO:115, the sequence of R6 comprises or consists of 16 to 30 consecutive nucleotides of SEQ ID NO:116 and the sequence of P6 comprises or consists of 20 to 30 consecutive nucleotides of SEQ ID NO:117;
g) the sequence of F7 comprises or consists of 16 to 30 consecutive nucleotides of SEQ ID NO: 128, the sequence of R7 comprises or consists of 16 to 30 consecutive nucleotides of SEQ ID NO: 129 and the sequence of P7 comprises or consists of 20 to 30 consecutive nucleotides of SEQ ID NO:130; and/or
h) the sequence of F8 comprises or consists of 16 to 30 consecutive nucleotides of SEQ ID NO: 139, the sequence of R8 comprises or consists of 16 to 30 consecutive nucleotides of SEQ ID NO: 140 and the sequence of P8 comprises or consists of 20 to 30 consecutive nucleotides of SEQ ID NO:141.

7. The method according to any one of claims 1 to 5, wherein
a) the sequence of F1 comprises or consists of SEQ ID NO: 15, the sequence of R1 comprises or consists of SEQ ID NO: 16 and the sequence of P1 comprises or consists of SEQ ID NO: 17; or the sequence of F1 comprises or consists of SEQ ID NO: 18, the sequence of R1 comprises or consists of SEQ ID NO: 19 and the sequence of P1 comprises or consists of SEQ ID NO:20; or the sequence of F1 comprises or consists of SEQ ID NO:21, the sequence of R1 comprises or consists of SEQ ID NO:22 and the sequence of P1 comprises or consists of SEQ ID NO:23; or the sequence of F1 comprises or consists of SEQ ID NO:24, the sequence of R1 comprises or consists of SEQ ID NO:25 and the sequence of P1 comprises or consists of SEQ ID NO:26;
b) the sequence of F2 comprises or consists of SEQ ID NO:38, the sequence of R2 comprises or consists of SEQ ID NO:39 and the sequence of P2 comprises or consists of SEQ ID NO:40; or the sequence of F2 comprises or consists of SEQ ID NO:41, the sequence of R2 comprises or consists of SEQ ID NO:42 and the sequence of P2 comprises or consists of SEQ ID NO:43; or the sequence of F2 comprises or consists of SEQ ID NO:44, the sequence of R2 comprises or consists of SEQ ID NO:45 and the sequence of P2 comprises or consists of SEQ ID NO:46;
c) the sequence of F3 comprises or consists of SEQ ID NO:59, the sequence of R3 comprises or consists of SEQ ID NO:60 and the sequence of P3 comprises or consists of SEQ ID NO:61; or the sequence of F3 comprises or consists of SEQ ID NO:62, the sequence of R3 comprises or consists of SEQ ID NO:63 and the sequence of P3 comprises or consists of SEQ ID NO:64; or the sequence of F3 comprises or consists of SEQ ID NO:65, the sequence of R3 comprises or consists of SEQ ID NO:66 or SEQ ID NO:67 and the sequence of P3 comprises or consists of SEQ ID NO:68;
d) the sequence of F4 comprises or consists of SEQ ID NO:73, the sequence of R4 comprises or consists of SEQ ID NO:74 and the sequence of P4 comprises or consists of SEQ ID NO:75; or the sequence of F4 comprises or consists of SEQ ID NO:76, the sequence of R4 comprises or consists of SEQ ID NO:77 and the sequence of P4 comprises or consists of SEQ ID NO:78; or the sequence of F4 comprises or consists of SEQ ID NO:83, the sequence of R4 comprises or consists of SEQ ID NO:84 and the sequence of P4 comprises or consists of SEQ ID NO:85; or the sequence of F4 comprises or consists of SEQ ID NO:90, the sequence of R4 comprises or consists of SEQ ID NO:91 and the sequence of P4 comprises or consists of SEQ ID NO:92;
e) the sequence of F5 comprises or consists of SEQ ID NO: 104, the sequence of R5 comprises or consists of SEQ ID NO: 105 and the sequence of P5 comprises or consists of SEQ ID NO: 106; or the sequence of F5 comprises or consists of SEQ ID NO: 107, the sequence of R5 comprises or consists of SEQ ID NO: 108 and the sequence of P5 comprises or consists of SEQ ID NO: 109; or the sequence of F5 comprises or consists of SEQ ID NO:110, the sequence of R5 comprises or consists of SEQ ID NO: 111 and the sequence of P5 comprises or consists of SEQ ID NO: 112;
f) the sequence of F6 comprises or consists of SEQ ID NO: 121, the sequence of R6 comprises or consists of SEQ ID NO: 122 and the sequence of P6 comprises or consists of SEQ ID NO: 123; or the sequence of F6 comprises or consists of SEQ ID NO: 124, the sequence of R6 comprises or consists of SEQ ID NO: 125 and the sequence of P6 comprises or consists of SEQ ID NO: 126;
g) the sequence of F7 comprises or consists of SEQ ID NO: 133 or SEQ ID NO: 136, the sequence of R7 comprises or consists of SEQ ID NO:134 and the sequence of P7 comprises or consists of SEQ ID NO: 135 or SEQ ID NO:137; and/or
h) the sequence of F8 comprises or consists of SEQ ID NO: 143, the sequence of R8 comprises or consists of SEQ ID NO: 144 and the sequence of P8 comprises or consists of SEQ ID NO:145 or SEQ ID NO: 146.

8. The method according to any one of claims 1 to 5, wherein
a) the sequence of F1 comprises or consists of SEQ ID NO: 15, the sequence of R1 comprises or consists of SEQ ID NO: 16 and the sequence of P1 comprises or consists of SEQ ID NO:17;
b) the sequence of F2 comprises or consists of SEQ ID NO:38, the sequence of R2 comprises or consists of SEQ ID NO:39 and the sequence of P2 comprises or consists of SEQ ID NO:40;
c) the sequence of F3 comprises or consists of SEQ ID NO:59, the sequence of R3 comprises or consists of SEQ ID NO:60 and the sequence of P3 comprises or consists of SEQ ID NO:61;
d) the sequence of F4 comprises or consists of SEQ ID NO:73, the sequence of R4 comprises or consists of SEQ ID NO:74 and the sequence of P4 comprises or consists of SEQ ID NO:75;
e) the sequence of F5 comprises or consists of SEQ ID NO: 104, the sequence of R5 comprises or consists of SEQ ID NO:105 and the sequence of P5 comprises or consists of SEQ ID NO:106;
f) the sequence of F6 comprises or consists of SEQ ID NO: 121, the sequence of R6 comprises or consists of SEQ ID NO:122 and the sequence of P6 comprises or consists of SEQ ID NO:123;
g) the sequence of F7 comprises or consists of SEQ ID NO: 133, the sequence of R7 comprises or consists of SEQ ID NO: 134 and the sequence of P7 comprises or consists of SEQ ID NO: 135; and/or
h) the sequence of F8 comprises or consists of SEQ ID NO: 143, the sequence of R8 comprises or consists of SEQ ID NO:144 and the sequence of P8 comprises or consists of SEQ ID NO:145.

9. An oligonucleotide set comprising
a) a forward primer F1 comprising or consisting of SEQ ID NO:15, a reverse primer R1 comprising or consisting of SEQ ID NO:16 and optionally a probe P1 comprising or consisting of SEQ ID NO:17;
b) a forward primer F2 comprising or consisting of SEQ ID NO:38, a reverse primer R2 comprising or consisting of SEQ ID NO:39 and optionally a probe P2 comprising or consisting of SEQ ID NO:40;
c) a forward primer F3 comprising or consisting of SEQ ID NO:59, a reverse primer R3 comprising or consisting of SEQ ID NO:60 and optionally a probe P3 comprising or consisting of SEQ ID NO:61;
d) a forward primer F4 comprising or consisting of SEQ ID NO:73, a reverse primer R4 comprising or consisting of SEQ ID NO:74 and optionally a probe P4 comprising or consisting of SEQ ID NO:75;
e) a forward primer F5 comprising or consisting of SEQ ID NO:104, a reverse primer R5 comprising or consisting of SEQ ID NO:105 and optionally a probe P5 comprising or consisting of SEQ ID NO:106;
f) a forward primer F6 comprising or consisting of SEQ ID NO:121, a reverse primer R6 comprising or consisting of SEQ ID NO:122 and optionally a probe P6 comprising or consisting of SEQ ID NO:123;
g) a forward primer F7 comprising or consisting of SEQ ID NO:133, a reverse primer R7 comprising or consisting of SEQ ID NO:134 and optionally a probe P7 comprising or consisting of SEQ ID NO:135; and/or
h) a forward primer F8 comprising or consisting of SEQ ID NO:143, a reverse primer R8 comprising or consisting of SEQ ID NO:144 and optionally a probe P8 comprising or consisting of SEQ ID NO:145.

10. A kit comprising an oligonucleotide set according to claim 9 and a component selected from the group consisting of a buffer, nucleotides and a DNA polymerase.

11. A use of the oligonucleotide set according to claim 9 or the kit according to claim 10 for determining in a sample the presence or absence of carbapenemase producing bacteria.

12. A method of diagnosis of an infection with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene; wherein the presence of a carbapenemase gene in the sample is indicative of an infection with carbapenemase producing bacteria.

13. An in vitro method for selecting a patient for treatment with a beta lactam antibacterial drug, preferably a carbapenem, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, selecting the patient for treatment with a beta lactam antibacterial drug, preferably a carbapenem.

14. A beta lactam antibacterial drug, preferably a carbapenem for use in a method of treatment of a bacterial infection in a patient that is not infected with carbapenemase producing bacteria, the method comprising
a) obtaining a sample of a patient;
b) determining in the sample the presence or absence of carbapenemase producing bacteria, comprising determining in said sample the presence or absence of one or more carbapenemase genes selected from the group consisting of an NDM carbapenemase gene, a KPC carbapenemase gene, a VIM carbapenemase gene, an IMP carbapenemase gene, an OXA-23-like carbapenemase gene, an OXA-48-like carbapenemase gene, an OXA-58-like carbapenemase gene and an OXA-40/24-like carbapenemase gene;
c) if the sample does not comprise carbapenemase producing bacteria, administering to the patient a beta lactam antibacterial drug, preferably a carbapenem.

15. The method of diagnosis according to claim 12, the in vitro method according to claim 13 or the beta lactam antibacterial drug for use according to claim 14, wherein step b) comprises determining the presence or absence of carbapenemase producing bacteria according to the method of any one of claims 1 to 9.


SEQUENCE LISTING

<110>  Universität Heidelberg

<120>  MULTIPLEX PCR FOR DETECTION OF CARBAPENEMASE GENES

<130>  789-38 EP

<160>  149

<170>  PatentIn version 3.5

<210>  1
<211>  410
<212>  DNA
<213>  Artificial Sequence

<220>
<223>  bacterial consensus

<400>  1
accgcccaga tcctcaactg gatcaagcag gagatcaacc tgccggtcgc gctggcggtg      60

gtgactcacg cgcatcagga caagatgggc ggtatgrrcg cgctgcatgc ggcggggatt     120

gcgacttatg ccaatgcgtt gtcgaaccag cttgccccgc aaraggggvt ggttgcggcg     180

caacacagcc tgactttcgc cgccaatggc tgggtcraac cagcaaccgc gcccaacttt     240

ggcccgctca aggtatttta ccccggcccc ggccacacca gtgacaatat caccgttggg     300

atcgacsgca ccgacatcgc ttttggtggc tgcctgatca aggacagcaa ggccaagtcg     360

ctcggcaatc tcgrtgatgc cgacactgag cactacgccg cgtcagygcg                410

<210>  2
<211>  131
<212>  DNA
<213>  Artificial Sequence

<220>
<223>  bacterial consensus

<400>  2
cggccacacc agtgacaata tcaccgttgg gatcgacsgc accgacatcg cttttggtgg      60

ctgcctgatc aaggacagca aggccaagtc gctcggcaat ctcgrtgatg ccgacactga     120

gcactacgcc g                                                          131

<210>  3
<211>  30
<212>  DNA
<213>  Artificial Sequence

<220>
<223>  bacterial consensus

<400>  3
cggccacacc agtgacaata tcaccgttgg                                       30

<210>  4
<211>  30
<212>  DNA
<213>  Artificial Sequence

<220>
<223>  bacterial consensus

<400>  4
cggcgtagtg ctcagtgtcg gcatcaycga                                        30


<210>  5
<211>  36
<212>  DNA
<213>  Artificial Sequence

<220>
<223>  bacterial consensus

<400>  5
ccgagcgact tggccttgct gtccttgatc aggcag                                 36


<210>  6
<211>  21
<212>  DNA
<213>  Artificial Sequence

<220>
<223>  bacterial consensus

<400>  6
cgcaacacag cctgactttc g                                                 21


<210>  7
<211>  32
<212>  DNA
<213>  Artificial Sequence

<220>
<223>  bacterial consensus

<400>  7
sgtcgatccc aacggtgata ttgtcactgg tg                                     32


<210>  8
<211>  39
<212>  DNA
<213>  Artificial Sequence

<220>
<223>  bacterial consensus

<400>  8
caaccgcgcc caactttggc ccgctcaagg tattttacc                              39


<210>  9
<211>  38

<212>  DNA
<213>  Artificial Sequence

<220>
<223>  bacterial consensus

<400>  9
cccagatcct caactggatc aagcaggaga tcaacctg                              38


<210>  10
<211>  35
<212>  DNA
<213>  Artificial Sequence

<220>
<223>  bacterial consensus

<400>  10
tggttcgaca acgcattggc ataagtcgca atccc                                 35


<210>  11
<211>  33
<212>  DNA
<213>  Artificial Sequence

<220>
<223>  bacterial consensus

<400>  11
cggtggtgac tcacgcgcat caggacaaga tgg                                   33


<210>  12
<211>  32
<212>  DNA
<213>  Artificial Sequence

<220>
<223>  bacterial consensus

<400>  12
caactttggc ccgctcaagg tattttaccc cg                                    32


<210>  13
<211>  23
<212>  DNA
<213>  Artificial Sequence

<220>
<223>  bacterial consensus

<400>  13
ggcgtagtgc tcagtgtcgg caa                                              23


<210>  14
<211>  35
<212>  DNA
<213>  Artificial Sequence

<220>
<223>  bacterial consensus

<400>  14
cgagattgcc gagcgacttg gccttgctgt ccttg                                35


<210>  15
<211>  20
<212>  DNA
<213>  Artificial Sequence

<220>
<223>  bacterial consensus

<400>  15
gccacaccag tgacaatatc                                                 20


<210>  16
<211>  17
<212>  DNA
<213>  Artificial Sequence

<220>
<223>  bacterial consensus

<400>  16
gtgctcagtg tcggcat                                                    17


<210>  17
<211>  24
<212>  DNA
<213>  Artificial Sequence

<220>
<223>  bacterial consensus

<400>  17
acttggcctt gctgtccttg atca                                            24


<210>  18
<211>  18
<212>  DNA
<213>  Artificial Sequence

<220>
<223>  bacterial consensus

<400>  18
gcaacacagc ctgacttt                                                   18


<210>  19
<211>  19
<212>  DNA
<213>  Artificial Sequence

<220>
<223>  bacterial consensus

<400>  19
tcgatcccaa cggtgatat                                                    19


<210>  20
<211>  23
<212>  DNA
<213>  Artificial Sequence

<220>
<223>  bacterial consensus

<400>  20
ccaactttgg cccgctcaag gta                                               23


<210>  21
<211>  19
<212>  DNA
<213>  Artificial Sequence

<220>
<223>  bacterial consensus

<400>  21
tcctcaactg gatcaagca                                                    19


<210>  22
<211>  20
<212>  DNA
<213>  Artificial Sequence

<220>
<223>  bacterial consensus

<400>  22
attggcataa gtcgcaatcc                                                   20


<210>  23
<211>  20
<212>  DNA
<213>  Artificial Sequence

<220>
<223>  bacterial consensus

<400>  23
tggtgactca cgcgcatcag                                                   20


<210>  24
<211>  20
<212>  DNA
<213>  Artificial Sequence

<220>
<223>  bacterial consensus

<400>  24
gcccgctcaa ggtattttac                                                   20

<210>  25
<211>  18
<212>  DNA
<213>  Artificial Sequence

<220>
<223>  bacterial consensus

<400>  25
tagtgctcag tgtcggca                                                    18


<210>  26
<211>  20
<212>  DNA
<213>  Artificial Sequence

<220>
<223>  bacterial consensus

<400>  26
agattgccga gcgacttggc                                                  20


<210>  27
<211>  418
<212>  DNA
<213>  Artificial Sequence

<220>
<223>  bacterial consensus

<400>  27
aacctcrtcg cggaaccatt cgctaaactc gaacaggact ttggcggctc catcggtgtg      60

tacgcgatrg ataccggytc aggcgcaact gtaagttacc gcgctgagga gcgcttccca     120

ctgtgcagct cattcaaggg ctttcttgct gccgctgtgc tggctcgcag ccagcagcag     180

gccggcttgc tggrcacacc catccgttac ggcaaaaayg cgctggttcb gdggtcaccc     240

atctcggaaa aatatctgac aacaggcatg acggtkkygg agctgtccgc ggccgccgtg     300

caatacagtg ataacgccgc cgccaatttg ttgctgaagg agttgggcgg cccggccrrr     360

ctgacggcct tcatgcgctc tatcggcgat accacgttcc gtctggacyg ctgggagc       418


<210>  28
<211>  161
<212>  DNA
<213>  Artificial Sequence

<220>
<223>  bacterial consensus

<400>  28
cccactgtgc agctcattca agggctttct tgctgccgct gtgctggctc gcagccagca      60

gcaggccggc ttgctggrca cacccatccg ttacggcaaa aaygcgctgg ttcbgdggtc     120

acccatctcg gaaaaatatc tgacaacagg catgacggtk k                         161

<210>  29
<211>  36
<212>  DNA
<213>  Artificial Sequence

<220>
<223>  bacterial consensus

<400>  29
cccactgtgc agctcattca agggctttct tgctgc                                36


<210>  30
<211>  33
<212>  DNA
<213>  Artificial Sequence

<220>
<223>  bacterial consensus

<400>  30
mmaccgtcat gcctgttgtc agatattttt ccg                                   33


<210>  31
<211>  41
<212>  DNA
<213>  Artificial Sequence

<220>
<223>  bacterial consensus

<400>  31
cttgctggrc acacccatcc gttacggcaa aaaygcgctg g                          41


<210>  32
<211>  32
<212>  DNA
<213>  Artificial Sequence

<220>
<223>  bacterial consensus

<400>  32
ctcrtcgcgg aaccattcgc taaactcgaa ca                                    32


<210>  33
<211>  40
<212>  DNA
<213>  Artificial Sequence

<220>
<223>  bacterial consensus

<400>  33
cagcggcagc aagaaagccc ttgaatgagc tgcacagtgg                            40


<210>  34

<211>  33
<212>  DNA
<213>  Artificial Sequence

<220>
<223>  bacterial consensus

<400>  34
actttggcgg ctccatcggt gtgtacgcga trg                                    33


<210>  35
<211>  26
<212>  DNA
<213>  Artificial Sequence

<220>
<223>  bacterial consensus

<400>  35
aaatatctga caacaggcat gacggt                                            26


<210>  36
<211>  33
<212>  DNA
<213>  Artificial Sequence

<220>
<223>  bacterial consensus

<400>  36
crgtccagac ggaacgtggt atcgccgata gag                                    33


<210>  37
<211>  35
<212>  DNA
<213>  Artificial Sequence

<220>
<223>  bacterial consensus

<400>  37
ccgccaattt gttgctgaag gagttgggcg gcccg                                  35


<210>  38
<211>  18
<212>  DNA
<213>  Artificial Sequence

<220>
<223>  bacterial consensus

<400>  38
cagctcattc aagggctt                                                     18


<210>  39
<211>  18
<212>  DNA
<213>  Artificial Sequence

<220>
<223>  bacterial consensus

<400>  39
cgtcatgcct gttgtcag                                                    18


<210>  40
<211>  21
<212>  DNA
<213>  Artificial Sequence

<220>
<223>  bacterial consensus

<400>  40
cacacccatc cgttacggca a                                                21


<210>  41
<211>  20
<212>  DNA
<213>  Artificial Sequence

<220>
<223>  bacterial consensus

<400>  41
cggaaccatt cgctaaactc                                                  20


<210>  42
<211>  20
<212>  DNA
<213>  Artificial Sequence

<220>
<223>  bacterial consensus

<400>  42
gaaagccctt gaatgagctg                                                  20


<210>  43
<211>  20
<212>  DNA
<213>  Artificial Sequence

<220>
<223>  bacterial consensus

<400>  43
actttggcgg ctccatcggt                                                  20


<210>  44
<211>  20
<212>  DNA
<213>  Artificial Sequence

<220>
<223>  bacterial consensus

<400>  44
atctgacaac aggcatgacg                                                   20


<210>  45
<211>  20
<212>  DNA
<213>  Artificial Sequence

<220>
<223>  bacterial consensus

<400>  45
gtccagacgg aacgtggtat                                                   20


<210>  46
<211>  20
<212>  DNA
<213>  Artificial Sequence

<220>
<223>  bacterial consensus

<400>  46
ttgctgaagg agttgggcgg                                                   20


<210>  47
<211>  176
<212>  DNA
<213>  Artificial Sequence

<220>
<223>  bacterial consensus

<400>  47
ggcgyrgtst acccrtccaa tggtctcatt gtccgtgatg gygatgagtt gcttttgatt      60

gatacagckt ggggtrcgaa aaacacagyg gcmcttctcg cggagattga raagcaaatt     120

ggacttccyg taacgcgtkc artctccacg cactttcatg acgaccgcgt cggcgg         176


<210>  48
<211>  153
<212>  DNA
<213>  Artificial Sequence

<220>
<223>  bacterial consensus

<400>  48
acccrtccaa tggtctcatt gtccgtgatg gygatgagtt gcttttgatt gatacagckt      60

ggggtrcgaa aaacacagyg gcmcttctcg cggagattga raagcaaatt ggacttccyg     120

taacgcgtkc artctccacg cactttcatg acg                                  153


<210>  49
<211>  31

<212>  DNA
<213>  Artificial Sequence

<220>
<223>  bacterial consensus

<400>  49
acccrtccaa tggtctcatt gtccgtgatg g                                    31


<210>  50
<211>  26
<212>  DNA
<213>  Artificial Sequence

<220>
<223>  bacterial consensus

<400>  50
cgtcatgaaa gtgcgtggag aytgma                                          26


<210>  51
<211>  42
<212>  DNA
<213>  Artificial Sequence

<220>
<223>  bacterial consensus

<400>  51
atggygatga gttgcttttg attgatacag cktggggtrc ga                        42


<210>  52
<211>  29
<212>  DNA
<213>  Artificial Sequence

<220>
<223>  bacterial consensus

<400>  52
ccrtccaatg gtctcattgt ccgtgatgg                                       29


<210>  53
<211>  29
<212>  DNA
<213>  Artificial Sequence

<220>
<223>  bacterial consensus

<400>  53
gcggtcgtca tgaaagtgcg tggagaytg                                       29


<210>  54
<211>  37
<212>  DNA
<213>  Artificial Sequence

<220>
<223>  bacterial consensus

<400>  54
atgagttgct tttgattgat acagcktggg gtrcgaa                                37


<210>  55
<211>  36
<212>  DNA
<213>  Artificial Sequence

<220>
<223>  bacterial consensus

<400>  55
gcggagattg araagcaaat tggacttccy gtaacg                                 36


<210>  56
<211>  24
<212>  DNA
<213>  Artificial Sequence

<220>
<223>  bacterial consensus

<400>  56
ggygatgcgt acgtygccac ycca                                              24


<210>  57
<211>  28
<212>  DNA
<213>  Artificial Sequence

<220>
<223>  bacterial consensus

<400>  57
crctcgatga gagyccttct agagartg                                          28


<210>  58
<211>  34
<212>  DNA
<213>  Artificial Sequence

<220>
<223>  bacterial consensus

<400>  58
cartctccac gcactttcat gacgaccgcg tcgg                                   34


<210>  59
<211>  19
<212>  DNA
<213>  Artificial Sequence

<220>
<223>  bacterial consensus

<400>  59
tccaatggtc tcattgtcc                                                    19


<210>  60
<211>  18
<212>  DNA
<213>  Artificial Sequence

<220>
<223>  bacterial consensus

<400>  60
catgaaagtg cgtggaga                                                     18


<210>  61
<211>  29
<212>  DNA
<213>  Artificial Sequence

<220>
<223>  bacterial consensus

<400>  61
atgagttgct tttgattgat acagcktgg                                         29


<210>  62
<211>  20
<212>  DNA
<213>  Artificial Sequence

<220>
<223>  bacterial consensus

<400>  62
aatggtctca ttgtccgtga                                                   20


<210>  63
<211>  20
<212>  DNA
<213>  Artificial Sequence

<220>
<223>  bacterial consensus

<400>  63
gtcatgaaag tgcgtggaga                                                   20


<210>  64
<211>  29
<212>  DNA
<213>  Artificial Sequence

<220>
<223>  bacterial consensus

<400>  64
atgagttgct tttgattgat acagcktgg                                         29

<210>  65
<211>  20
<212>  DNA
<213>  Artificial Sequence

<220>
<223>  bacterial consensus

<400>  65
garaagcaaa ttggacttcc                                                   20


<210>  66
<211>  17
<212>  DNA
<213>  Artificial Sequence

<220>
<223>  bacterial consensus

<400>  66
gatgcgtacg tygccac                                                      17


<210>  67
<211>  21
<212>  DNA
<213>  Artificial Sequence

<220>
<223>  bacterial consensus

<400>  67
ctcgatgaga gyccttctag a                                                 21


<210>  68
<211>  24
<212>  DNA
<213>  Artificial Sequence

<220>
<223>  bacterial consensus

<400>  68
tctccacgca ctttcatgac gacc                                              24


<210>  69
<211>  201
<212>  DNA
<213>  Artificial Sequence

<220>
<223>  bacterial consensus

<400>  69
ggyggaatag agtggcttaa ttctcaatct atyccmacrt atgcatctga attaacaaat      60

gaacttctta aaaaagrcgg taargtacaa gctaaawatt catttrgcgg agbyagctat     120

tggytagtta akaawaagat tgaarttttt tatcctggyy cagggcacrc tccagataac      180

gtagtggttt ggctrcctga a                                                201

<210>  70
<211>  30
<212>  DNA
<213>  Artificial Sequence

<220>
<223>  bacterial consensus

<400>  70
ggyggaatag agtggcttaa ttctcaatct                                        30

<210>  71
<211>  32
<212>  DNA
<213>  Artificial Sequence

<220>
<223>  bacterial consensus

<400>  71
ttcaggyagc caaaccacta cgttatctgg ag                                     32

<210>  72
<211>  41
<212>  DNA
<213>  Artificial Sequence

<220>
<223>  bacterial consensus

<400>  72
ccmacrtatg catctgaatt aacaaatgaa cttcttaaaa a                           41

<210>  73
<211>  22
<212>  DNA
<213>  Artificial Sequence

<220>
<223>  bacterial consensus

<400>  73
ggaatagagt ggcttaattc tc                                                22

<210>  74
<211>  21
<212>  DNA
<213>  Artificial Sequence

<220>
<223>  bacterial consensus

<400>  74
gccaaaccac tacgttatct k                                                 21

<210>  75
<211>  28
<212>  DNA
<213>  Artificial Sequence

<220>
<223>  bacterial consensus

<400>  75
atgcrtctga attaacaaat garcttct                                          28


<210>  76
<211>  25
<212>  DNA
<213>  Artificial Sequence

<220>
<223>  bacterial consensus

<400>  76
ggtggaatag agtggcttaa ttctc                                             25


<210>  77
<211>  24
<212>  DNA
<213>  Artificial Sequence

<220>
<223>  bacterial consensus

<400>  77
gccaaaccac tacgttatct kgag                                              24


<210>  78
<211>  35
<212>  DNA
<213>  Artificial Sequence

<220>
<223>  bacterial consensus

<400>  78
cccacgtatg crtctgaatt aacaaatgar cttct                                  35


<210>  79
<211>  226
<212>  DNA
<213>  Artificial Sequence

<220>
<223>  bacterial consensus

<400>  79
gwtttgyagc attgctaccg cagcagagyc tttgccagat ttaaaaattg aaaarcttga      60

wgaaggcgtt tatgttcata cttcgtttra agaagttaac rggtggggck ttkttcctaa     120

acatggtttg gtkgttcttg taratgctga rgcttayhta attgacactc catytacggc     180

taaagatact gaaaagttag kcacttggtt tgtggarcgt ggctat                    226

<210>  80
<211>  31
<212>  DNA
<213>  Artificial Sequence

<220>
<223>  bacterial consensus

<400>  80
ttgyagcatt gctaccgcag cagagycttt g                                     31

<210>  81
<211>  32
<212>  DNA
<213>  Artificial Sequence

<220>
<223>  bacterial consensus

<400>  81
acgytccaca aaccaagtga ctaacttttc ag                                    32

<210>  82
<211>  32
<212>  DNA
<213>  Artificial Sequence

<220>
<223>  bacterial consensus

<400>  82
wgaaggcgtt tatgttcata cttcgtttra ag                                    32

<210>  83
<211>  18
<212>  DNA
<213>  Artificial Sequence

<220>
<223>  bacterial consensus

<400>  83
attgctaccg cagcagag                                                    18

<210>  84
<211>  22
<212>  DNA
<213>  Artificial Sequence

<220>
<223>  bacterial consensus

<400>  84
tccacaaacc aagtgactaa ct                                               22

<210>  85
<211>  26
<212>  DNA
<213>  Artificial Sequence

<220>
<223>  bacterial consensus

<400>  85
aaggcgttta tgttcatact tcgttt                                          26


<210>  86
<211>  178
<212>  DNA
<213>  Artificial Sequence

<220>
<223>  bacterial consensus

<400>  86
cctatctrat tgacactcca tttactgcta cagayactga aaarttagtc aattggtttg      60

tggagcgcgg ctatraaatc aaaggcacta tttcmtcaca tttccatagc gacagcacag     120

grggaataga gtggcttaat tctcaatcta ttcccacgta tgcatctgaa ttaacaaa       178


<210>  87
<211>  36
<212>  DNA
<213>  Artificial Sequence

<220>
<223>  bacterial consensus

<400>  87
atctrattga cactccattt actgctacag yactga                               36


<210>  88
<211>  37
<212>  DNA
<213>  Artificial Sequence

<220>
<223>  bacterial consensus

<400>  88
tgttaattca gatgcatacg tgggaataga ttgagaa                              37


<210>  89
<211>  30
<212>  DNA
<213>  Artificial Sequence

<220>
<223>  bacterial consensus

<400>  89
ttagtcaatt ggtttgtgga gcgcggctat                                      30

<210>  90
<211>  23
<212>  DNA
<213>  Artificial Sequence

<220>
<223>  bacterial consensus

<400>  90
tgacactcca tttactgcta cag                                              23


<210>  91
<211>  20
<212>  DNA
<213>  Artificial Sequence

<220>
<223>  bacterial consensus

<400>  91
cagatgcata cgtgggaata                                                  20


<210>  92
<211>  23
<212>  DNA
<213>  Artificial Sequence

<220>
<223>  bacterial consensus

<400>  92
gtcaattggt ttgtggagcg cgg                                              23


<210>  93
<211>  460
<212>  DNA
<213>  Artificial Sequence

<220>
<223>  bacterial consensus

<400>  93
tytttctggt tgtacggttc agcataattt aataaatgaa acccmgagtc agattgttca      60

aggacataat caggtgattc atcaatactt tgatgaaara aacacctcag gtgygctggt     120

yattcaaaca gataaaaaaa ttaatytrta tggtaatgct ctaagccgcg caaatacaga     180

atatgtgcca gcctctacat ttaaaatgtt gaatgccctg atcggattgg agaaccagaa     240

arcggatatt aatgaaatat ttaaatggaa gggcgagaaa aggtyattta ccrcttggga     300

aaaagacatg acactaggag aagccatgaa gctttctgca gtcccagtct atcaggaact     360

tgcrmgacgt atcggtcttg atctcatgca aaaagaagta raacgtattg rtttcggtaa     420

tgctgaaatt ggacagcagg ttgayaattt ctggttgrta                           460

<210>  94
<211>  204
<212>  DNA
<213>  Artificial Sequence

<220>
<223>  bacterial consensus

<400>  94
tattaatgaa atatttaaat ggaagggcga gaaaaggtya tttaccrctt gggaaaaaga      60

catgacacta ggagaagcca tgaagctttc tgcagtccca gtctatcagg aacttgcrmg      120

acgtatcggt cttgatctca tgcaaaaaga agtaraacgt attgrtttcg gtaatgctga      180

aattggacag caggttgaya attt                                              204


<210>  95
<211>  43
<212>  DNA
<213>  Artificial Sequence

<220>
<223>  bacterial consensus

<400>  95
tattaatgaa atatttaaat ggaagggcga gaaaaggtya ttt                         43


<210>  96
<211>  39
<212>  DNA
<213>  Artificial Sequence

<220>
<223>  bacterial consensus

<400>  96
aaattrtcaa cctgctgtcc aatttcagca ttaccgaaa                              39


<210>  97
<211>  48
<212>  DNA
<213>  Artificial Sequence

<220>
<223>  bacterial consensus

<400>  97
ctaggagaag ccatgaagct ttctgcagtc ccagtctatc aggaactt                    48


<210>  98
<211>  30
<212>  DNA
<213>  Artificial Sequence

<220>
<223>  bacterial consensus

<400>  98
tytttctggt tgtacggttc agcataattt                                        30


<210>  99
<211>  41
<212>  DNA
<213>  Artificial Sequence

<220>
<223>  bacterial consensus

<400>  99
tgggactgca gaaagcttca tggcttctcc tagtgtcatg t                           41


<210>  100
<211>  35
<212>  DNA
<213>  Artificial Sequence

<220>
<223>  bacterial consensus

<400>  100
tgttcaagga cataatcagg tgattcatca atact                                  35


<210>  101
<211>  36
<212>  DNA
<213>  Artificial Sequence

<220>
<223>  bacterial consensus

<400>  101
ctctaagccg cgcaaataca gaatatgtgc cagcct                                 36


<210>  102
<211>  41
<212>  DNA
<213>  Artificial Sequence

<220>
<223>  bacterial consensus

<400>  102
tgggactgca gaaagcttca tggcttctcc tagtgtcatg t                           41


<210>  103
<211>  40
<212>  DNA
<213>  Artificial Sequence

<220>
<223>  bacterial consensus

<400>  103
aaatgttgaa tgccctgatc ggattggaga accagaaarc                             40

<210>  104
<211>  18
<212>  DNA
<213>  Artificial Sequence

<220>
<223>  bacterial consensus

<400>  104
taaatggaag ggcgagaa                                                    18


<210>  105
<211>  19
<212>  DNA
<213>  Artificial Sequence

<220>
<223>  bacterial consensus

<400>  105
acctgctgtc caatttcag                                                   19


<210>  106
<211>  26
<212>  DNA
<213>  Artificial Sequence

<220>
<223>  bacterial consensus

<400>  106
ccatgaagct ttctgcagtc ccagtc                                           26


<210>  107
<211>  18
<212>  DNA
<213>  Artificial Sequence

<220>
<223>  bacterial consensus

<400>  107
tctggttgta cggttcag                                                    18


<210>  108
<211>  19
<212>  DNA
<213>  Artificial Sequence

<220>
<223>  bacterial consensus

<400>  108
cggcttagag cattaccat                                                   19


<210>  109
<211>  27

<212>  DNA
<213>  Artificial Sequence

<220>
<223>  bacterial consensus

<400>  109
tcaaggacat aatcaggtga ttcatca                                          27


<210>  110
<211>  19
<212>  DNA
<213>  Artificial Sequence

<220>
<223>  bacterial consensus

<400>  110
gccgcgcaaa tacagaata                                                   19


<210>  111
<211>  20
<212>  DNA
<213>  Artificial Sequence

<220>
<223>  bacterial consensus

<400>  111
gcagaaagct tcatggcttc                                                  20


<210>  112
<211>  28
<212>  DNA
<213>  Artificial Sequence

<220>
<223>  bacterial consensus

<400>  112
gttgaatgcc ctgatcggat tggagaac                                         28


<210>  113
<211>  238
<212>  DNA
<213>  Artificial Sequence

<220>
<223>  bacterial consensus

<400>  113
tgccwgcggt agcaaaggaa tggcaagaaa acaaaagttg gaatgctcac tttactgaac      60

ataaatcaca gggcgtagtt gtgctctgga atgagaataa gcagcaagga tttaccaata     120

atcttaaacg ggcgaaccaa gcatttttac ccgcatctac ctttaaaatt cccaatagct     180

tgatcgccct cgatttgggc gtggttaagg atgaacacca agtctttaag tgggatgg       238

<210>  114
<211>  172
<212>  DNA
<213>  Artificial Sequence

<220>
<223>  bacterial consensus

<400>  114
gaacataaat cacagggcgt agttgtgctc tggaatgaga ataagcagca aggatttacc      60

aataatctta aacgggcgaa ccaagcattt ttacccgcat ctacctttaa aattcccaat     120

agcttgatcg ccctcgattt gggcgtggtt aaggatgaac accaagtctt ta             172


<210>  115
<211>  44
<212>  DNA
<213>  Artificial Sequence

<220>
<223>  bacterial consensus

<400>  115
gaacataaat cacagggcgt agttgtgctc tggaatgaga ataa                       44


<210>  116
<211>  39
<212>  DNA
<213>  Artificial Sequence

<220>
<223>  bacterial consensus

<400>  116
taaagacttg gtgttcatcc ttaaccacgc ccaaatcga                             39


<210>  117
<211>  43
<212>  DNA
<213>  Artificial Sequence

<220>
<223>  bacterial consensus

<400>  117
ttaccaataa tcttaaacgg gcgaaccaag catttttacc cgc                        43


<210>  118
<211>  36
<212>  DNA
<213>  Artificial Sequence

<220>
<223>  bacterial consensus

<400>  118
gcggtagcaa aggaatggca agaaaacaaa agttgg                                36

<210>  119
<211>  36
<212>  DNA
<213>  Artificial Sequence

<220>
<223>  bacterial consensus

<400>  119
taaaggtaga tgcgggtaaa aatgcttggt tcgccc                                36


<210>  120
<211>  46
<212>  DNA
<213>  Artificial Sequence

<220>
<223>  bacterial consensus

<400>  120
cataaatcac agggcgtagt tgtgctctgg aatgagaata agcagc                    46


<210>  121
<211>  17
<212>  DNA
<213>  Artificial Sequence

<220>
<223>  bacterial consensus

<400>  121
agggcgtagt tgtgctc                                                     17


<210>  122
<211>  20
<212>  DNA
<213>  Artificial Sequence

<220>
<223>  bacterial consensus

<400>  122
gtgttcatcc ttaaccacgc                                                  20


<210>  123
<211>  23
<212>  DNA
<213>  Artificial Sequence

<220>
<223>  bacterial consensus

<400>  123
tcttaaacgg gcgaaccaag cat                                              23


<210>  124

<211>  20
<212>  DNA
<213>  Artificial Sequence

<220>
<223>  bacterial consensus

<400>  124
agcaaaggaa tggcaagaaa                                                    20


<210>  125
<211>  20
<212>  DNA
<213>  Artificial Sequence

<220>
<223>  bacterial consensus

<400>  125
gatgcgggta aaaatgcttg                                                    20


<210>  126
<211>  23
<212>  DNA
<213>  Artificial Sequence

<220>
<223>  bacterial consensus

<400>  126
cagggcgtag ttgtgctctg gaa                                                23


<210>  127
<211>  181
<212>  DNA
<213>  Artificial Sequence

<220>
<223>  bacterial consensus

<400>  127
gtatatcaag aattggcacg tcgtattggt ccaagcttaa tgcaaagtga attgcaacgt        60

attggttatg gcaatatgca aatrggcacg gaagttgatc aattttggtt raaagggcct       120

ttgacaatta cacctataca agaagtaaag tttgtgtatg atttagccca agggcaattg       180

c                                                                       181


<210>  128
<211>  41
<212>  DNA
<213>  Artificial Sequence

<220>
<223>  bacterial consensus

<400>  128
tatatcaaga attggcacgt cgtattggtc caagcttaat g                            41

<210>  129
<211>  36
<212>  DNA
<213>  Artificial Sequence

<220>
<223>  bacterial consensus

<400>  129
gcaattgccc ttgggctaaa tcatacacaa acttta                                36


<210>  130
<211>  45
<212>  DNA
<213>  Artificial Sequence

<220>
<223>  bacterial consensus

<400>  130
ttaatgcaaa gtgaattgca acgtattggt tatggcaata tgcaa                      45


<210>  131
<211>  42
<212>  DNA
<213>  Artificial Sequence

<220>
<223>  bacterial consensus

<400>  131
tccaagctta atgcaaagtg aattgcaacg tattggttat gg                         42


<210>  132
<211>  47
<212>  DNA
<213>  Artificial Sequence

<220>
<223>  bacterial consensus

<400>  132
caaatrggca cggaagttga tcaattttgg ttraaagggc ctttgac                    47


<210>  133
<211>  18
<212>  DNA
<213>  Artificial Sequence

<220>
<223>  bacterial consensus

<400>  133
attggcacgt cgtattgg                                                    18


<210>  134

<211>  19
<212>  DNA
<213>  Artificial Sequence

<220>
<223>  bacterial consensus

<400>  134
gcccttgggc taaatcata                                                    19


<210>  135
<211>  28
<212>  DNA
<213>  Artificial Sequence

<220>
<223>  bacterial consensus

<400>  135
agtgaattgc aacgtattgg ttatggca                                          28


<210>  136
<211>  20
<212>  DNA
<213>  Artificial Sequence

<220>
<223>  bacterial consensus

<400>  136
atgcaaagtg aattgcaacg                                                   20


<210>  137
<211>  27
<212>  DNA
<213>  Artificial Sequence

<220>
<223>  bacterial consensus

<400>  137
ggcacggaag ttgatcaatt ttggttr                                           27


<210>  138
<211>  234
<212>  DNA
<213>  Artificial Sequence

<220>
<223>  bacterial consensus

<400>  138
agaaagatat gactttaggt gaggcaatgg cattgtcagc agttccagta tatcaagagc      60

ttgcaagacg gactggccta gagctaatgc agaaagaagt aaagcgggtt aattttggaa     120

atacaaatat tggaacacag gtcgataatt tttggttagt tggccccctt aaaattacac     180

cagtacaaga agttaatttt gccgatgacc ttgcacataa ccgattacct ttta           234

<210>  139
<211>  38
<212>  DNA
<213>  Artificial Sequence

<220>
<223>  bacterial consensus

<400>  139
agaaagatat gactttaggt gaggcaatgg cattgtca                                38


<210>  140
<211>  43
<212>  DNA
<213>  Artificial Sequence

<220>
<223>  bacterial consensus

<400>  140
taaaaggtaa tcggttatgt gcaaggtcat cggcaaaatt aac                          43


<210>  141
<211>  45
<212>  DNA
<213>  Artificial Sequence

<220>
<223>  bacterial consensus

<400>  141
tcaagagctt gcaagacgga ctggcctaga gctaatgcag aaaga                        45


<210>  142
<211>  48
<212>  DNA
<213>  Artificial Sequence

<220>
<223>  bacterial consensus

<400>  142
tcagcagttc cagtatatca agagcttgca agacggactg gcctagag                     48


<210>  143
<211>  20
<212>  DNA
<213>  Artificial Sequence

<220>
<223>  bacterial consensus

<400>  143
tgactttagg tgaggcaatg                                                    20


<210>  144

<211>  20
<212>  DNA
<213>  Artificial Sequence

<220>
<223>  bacterial consensus

<400>  144
gttatgtgca aggtcatcgg                                                   20


<210>  145
<211>  27
<212>  DNA
<213>  Artificial Sequence

<220>
<223>  bacterial consensus

<400>  145
tgcaagacgg actggcctag agctaat                                           27


<210>  146
<211>  27
<212>  DNA
<213>  Artificial Sequence

<220>
<223>  bacterial consensus

<400>  146
agtatatcaa gagcttgcaa gacggac                                           27


<210>  147
<211>  19
<212>  DNA
<213>  Artificial Sequence

<220>
<223>  bacterial consensus

<400>  147
tcctacggga ggcagcagt                                                    19


<210>  148
<211>  26
<212>  DNA
<213>  Artificial Sequence

<220>
<223>  bacterial consensus

<400>  148
ggactaccag ggtatctaat cctgtt                                            26


<210>  149
<211>  23
<212>  DNA
<213>  Artificial Sequence

<220>
<223>  bacterial consensus

<400>  149
cgtattaccg cggctgctgg cac                                              23